# EUROPEAN PATENT APPLICATION

(11) **EP 4 130 035 A1**
(43) Date of publication of application: **08.02.2023**
(21) Application number: 21780300.6
(22) Date of filing: 30.03.2021
(51) Int. Cl.: C07K 16/10, C07K 19/00, C12N 15/13, A61K 39/42, A61P 31/14

(54) **ANTIBODY AND FUSION PROTEIN FOR TREATING CORONAVIRUSES AND USE THEREOF**

(30) Priority: 31.03.2020 CN 202010244894; 13.04.2020 CN 202010287037; 13.04.2020 CN 202010286986; 27.04.2020 CN 202010344572; 27.04.2020 CN 202010344084; 06.08.2020 CN 202010784365
(71) Applicant: Bio-Thera Solutions, Ltd., Guangzhou, Guangdong 510530 (CN)
(72) Inventor: ZHANG, Hui, Guangzhou, Guangdong 510530 (CN); WAMG, Zhiwei, Guangzhou, Guangdong 510530 (CN); CHEN, Junyou, Guangzhou, Guangdong 510530 (CN); CHEN, Zhencheng, Guangzhou, Guangdong 510530 (CN); LI, Chuang, Guangzhou, Guangdong 510530 (CN); YAN, Qingwei, Guangzhou, Guangdong 510530 (CN); ZHENG, Dandan, Guangzhou, Guangdong 510530 (CN); YANG, Shaowei, Guangzhou, Guangdong 510530 (CN); LI, Jiaping, Guangzhou, Guangdong 510530 (CN); QIN, Chao, Guangzhou, Guangdong 510530 (CN); HUANG, Xianming, Guangzhou, Guangdong 510530 (CN); LI, Shengfeng, Guangzhou, Guangdong 510530 (CN); HUANG, Haohui, Guangzhou, Guangdong 510530 (CN); SU, Ziqi, Guangzhou, Guangdong 510530 (CN)
(74) Representative: Rimini, Rebecca
(86) International application number: PCT/CN2021/084069
(87) International publication number: WO 2021/197340

(57) **Abstract**

The present invention provides an antibody and a fusion protein for treating coronaviruses and use thereof. The 6-HB interference polypeptide in the fusion protein of the present invention and a part of an antibody or an antigen-binding fragment work synergistically to prevent fusion of SARS-CoV or SARS-CoV-2 virus particles with cells, and mediate phagocytosis and clearance of the virus particles by immune cells.

## Description

### TECHNICAL FIELD

The present invention belongs to field of biotechnology, and particularly relates to an antibody and a fusion protein for treating coronaviruses and use thereof.

### BACKGROUND

Coronaviruses are nonsegmented single-stranded positive-strand RNA viruses. According to serotype and genomic characteristics, the *coronaviridae* subfamily is divided into four genera: α, β, γ and δ. Coronaviruses are named for the corolla-like spike protruding all around from the viral envelope. The novel coronavirus (SARS-CoV-2 or 2019-nCoV) discovered in 2019 belongs to the β genus and has envelope, and the particles are round or oval and often multiform with a diameter of 60-140 nm. Current studies show that SARS-CoV-2 has a high homology with SARS-CoV.

The novel coronavirus pneumonia (COVID-19) is mainly transmitted through the respiratory tract, and it may also be transmitted by contact. People are generally susceptible to this disease, the old and people with underlying diseases suffer from relatively severe illness after the infection, and cases of children and infants are also present. Based on current epidemiological investigations, the latency period of the COVID-19 is generally 1 to 14 days, mostly 3 to 7 days. The main clinical symptoms of the infected patients are fever, hypodynamia and dry cough, and such symptoms of upper respiratory tract as nasal obstruction and running nose are rare. In the early stage of the disease, the total number of leucocytes of the patients is normal or reduced, or the number of lymphocytes is reduced, and the liver enzyme, the muscle enzyme and the myoglobin are increased in some patients. In the early stage, the chest imaging shows multiple small patchy shadows and interstitial changes, more apparent in the peripheral zone of lungs. As the disease progresses, imaging shows multiple ground-glass opacities and infiltration in both lungs. In severe cases, pulmonary consolidation may occur and gradually difficulty in breathing occurs, and if the situation is severe, there will be Acute Respiratory Distress Syndrome (ARDS), shock and injuries and dysfunctions of various tissues, including lung tissues, heart and kidney. The prognosis for most patients with mild infection is good, and patients with severe infection are often critically ill or would even die.

Recently, fundamental, clinical and epidemiological studies on COVID-19 have been delivered or published, but there is still a lack of effective therapeutic drugs.

### SUMMARY

The present invention provides an antibody or an antigen-binding fragment having high affinity for the spike protein of SARS-CoV and SARS-CoV-2 and a fusion protein comprising the antibody or the antigen-binding fragment.

The present invention provides an antibody or an antigen-binding fragment having high affinity for the spike protein of SARS-CoV and SARS-CoV-2. These antibodies can specifically bind to the spike protein, thus preventing binding of viral particles to cells and mediating phagocytosis and clearance of the virus particles by immune cells. These antibodies may be used for treating or ameliorating SARS and COVID-19, and also for diagnosing SARS and COVID-19.

Through the binding of the spike protein (S protein) on the surface of SARS-CoV or SARS-CoV-2 to angiotensin converting enzyme 2 (ACE2) on the surface of lung epithelial cells, SARS-CoV or SARS-CoV-2 enters into the cells and use the cells to synthesize new virus particles; the new virus particles are released outside the cells and then infect surrounding normal cells in the same manner. The anti-spike protein antibody can block the binding of the spike protein to ACE2 and further block virus from entering cells, thus playing a role of antiviral. The antibody of the present invention may also mediate immune cells to phagocytose and clear virus.

In some embodiments, provided is an antibody or an antigen-binding fragment specifically binding a spike protein and comprising:
(a) a VH CDR1 comprising an amino acid sequence set forth in SEQ ID NO: 1 or having a variant with a substitution, deletion or insertion at a single site; (b) a VH CDR2 comprising an amino acid sequence set forth in SEQ ID NO: 2 or having a variant with a substitution, deletion or insertion at a single site; and/or (c) a VH CDR3 comprising an amino acid sequence set forth in any one of SEQ ID NOs: 3-38 or 105 or having a variant with a substitution, deletion or insertion at a single site.

In some embodiments, provided is an antibody or an antigen-binding fragment specifically binding a spike protein and comprising:
(a) a VH CDR1 comprising an amino acid sequence set forth in SEQ ID NO: 1 or having a variant with a substitution, deletion or insertion at a single site; (b) a VH CDR2 comprising an amino acid sequence set forth in SEQ ID NO: 2 or having a variant with a substitution, deletion or insertion at a single site; and (c) a VH CDR3 comprising an amino acid sequence set forth in any one of SEQ ID NOs: 3-38 or 105 or having a variant with a substitution, deletion or insertion at a single site.

In some embodiments, provided is an antibody or an antigen-binding fragment specifically binding a spike protein and comprising:
(a) a VH CDR1 comprising an amino acid sequence set forth in SEQ ID NO: 1; (b) a VH CDR2 comprising an amino acid sequence set forth as SEQ ID NO: 2; and (c) a VH CDR3 comprising an amino acid sequence set forth in any one of SEQ ID NOs: 3-38 or 105.

In some embodiments, provided is an antibody or an antigen-binding fragment specifically binding a spike protein and comprising:
(a) a VH CDR1 comprising an amino acid sequence set forth in SEQ ID NO: 1 or having a variant with a substitution, deletion or insertion at a single site; (b) a VH CDR2 comprising an amino acid sequence set forth in SEQ ID NO: 2 or having a variant with a substitution, deletion or insertion at a single site; (c) a VH CDR3 comprising an amino acid sequence set forth in any one of SEQ ID NOs: 3-38 or 105 or having a variant with a substitution, deletion or insertion at a single site; (d) a VL CDR1 comprising an amino acid sequence set forth in SEQ ID NO: 39 or having a variant with a substitution, deletion or insertion at a single site; (e) a VL CDR2 comprising an amino acid sequence set forth in SEQ ID NO: 40 or having a variant with a substitution, deletion or insertion at a single site; and/or (f) a VL CDR3 comprising an amino acid sequence set forth in SEQ ID NO: 41 or having a variant with a substitution, deletion or insertion at a single site.

In some embodiments, the antibody or the antigen-binding fragment specifically binds a spike protein and comprises:
(a) a VH CDR1 comprising an amino acid sequence set forth in SEQ ID NO: 1 or having a variant with a substitution, deletion or insertion at a single site; (b) a VH CDR2 comprising an amino acid sequence set forth in SEQ ID NO: 2 or having a variant with a substitution, deletion or insertion at a single site; (c) a VH CDR3 comprising an amino acid sequence set forth in any one of SEQ ID NOs: 3-38 or 105 or having a variant with a substitution, deletion or insertion at a single site; (d) a VL CDR1 comprising an amino acid sequence set forth in SEQ ID NO: 39 or having a variant with a substitution, deletion or insertion at a single site; (e) a VL CDR2 comprising an amino acid sequence set forth in SEQ ID NO: 40 or having a variant with a substitution, deletion or insertion at a single site; and (f) a VL CDR3 comprising an amino acid sequence set forth in SEQ ID NO: 41 or having a variant with a substitution, deletion or insertion at a single site.

In some embodiments, a variant with a substitution is a variant with a conservative amino acid substitution.

In some embodiments, the antibody or the antigen-binding fragment specifically binds to a spike protein, and the antibody or the antigen-binding fragment at least comprises one, two, three, four, five, or all of the VH CDR1 set forth in SEQ ID NO: 1, the VH CDR2 set forth in SEQ ID NO: 2, the VH CDR3 set forth in any one of SEQ ID NOs: 3-38 or 105, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in any one of SEQ ID NOs: 3-38 or 105, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 3, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 4, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 5, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 6, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 7, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 8, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 9, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 10, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 11, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 12, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 13, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 14, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 15, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 16, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 17, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 18, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 19, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 20, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 21, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 22, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 23, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 24, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 25, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 26, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 27, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 28, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 29, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 30, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 31, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 32, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 33, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 34, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 35, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 36, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 37, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 38, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 105, the VL CDR1 set forth in SEQ ID NO: 39, the VL CDR2 set forth in SEQ ID NO: 40, and the VL CDR3 set forth in SEQ ID NO: 41.

In some embodiments, framework regions of a heavy chain variable region of the antibody or the antigen-binding fragment comprise a heavy chain FR1, a heavy chain FR2, a heavy chain FR3 and a heavy chain FR4, and the heavy chain FR1 of the antibody or the antigen-binding fragment comprises a sequence set forth in SEQ ID NO: 42, a sequence having at least 90% identity to the sequence set forth in SEQ ID NO: 42, or an amino acid sequence having one or more conservative amino acid substitutions as compared to the sequence set forth in SEQ ID NO: 42; and/or
the heavy chain FR2 of the antibody or the antigen-binding fragment comprises a sequence set forth in SEQ ID NO: 43, a sequence having at least 90% identity to the sequence set forth in SEQ ID NO: 43, or an amino acid sequence having one or more conservative amino acid substitutions as compared to the sequence set forth in SEQ ID NO: 43; and/or
the heavy chain FR3 of the antibody or the antigen-binding fragment comprises a sequence set forth in SEQ ID NO: 44, a sequence having at least 90% identity to the sequence set forth in SEQ ID NO: 44, or an amino acid sequence having one or more conservative amino acid substitutions as compared to the sequence set forth in SEQ ID NO: 44; and/or
the heavy chain FR4 of the antibody or the antigen-binding fragment comprises a sequence set forth in SEQ ID NO: 45, a sequence having at least 90% identity to the sequence set forth in SEQ ID NO: 45, or an amino acid sequence having one or more conservative amino acid substitutions as compared to the sequence set forth in SEQ ID NO: 45.

In some embodiments, the heavy chain FR1 comprises a sequence set forth in SEQ ID NO: 42, a sequence having at least 90% identity to the sequence set forth in SEQ ID NO: 42, or an amino acid sequence having one or more conservative amino acid substitutions as compared to the sequence set forth in SEQ ID NO: 42; the heavy chain FR2 comprises a sequence set forth in SEQ ID NO: 43, a sequence having at least 90% identity to the sequence set forth in SEQ ID NO: 43, or an amino acid sequence having one or more conservative amino acid substitutions as compared to the sequence set forth in SEQ ID NO: 43; the heavy chain FR3 comprises a sequence set forth in SEQ ID NO: 44, a sequence having at least 90% identity to the sequence set forth in SEQ ID NO: 44, or an amino acid sequence having one or more conservative amino acid substitutions as compared to the sequence set forth in SEQ ID NO: 44; the heavy chain FR4 comprises a sequence set forth in SEQ ID NO: 45, a sequence having at least 90% identity to the sequence set forth in SEQ ID NO: 45, or an amino acid sequence having one or more conservative amino acid substitutions as compared to the sequence set forth in SEQ ID NO: 45. In some embodiments, the one or more conservative amino acid substitutions are about 1, about 2, or about 3 conservative amino acid substitutions.

In some embodiments, the heavy chain FR1 comprises a sequence set forth in SEQ ID NO: 42, the heavy chain FR2 comprises a sequence set forth in SEQ ID NO: 43, the heavy chain FR3 comprises a sequence set forth in SEQ ID NO: 44, and the heavy chain FR4 comprises a sequence set forth in SEQ ID NO: 45. In some embodiments, the heavy chain variable region comprises the structure FR1-VH CDR1-FR2-VH CDR2-FR3-VH CDR3-FR4.

In some embodiments, provided is an antibody or an antigen-binding fragment specifically binding to a spike protein and comprising: (a) a VH CDR1 comprising an amino acid sequence set forth in SEQ ID NO: 60 or having a variant with a substitution, deletion or insertion at a single site; (b) a VH CDR2 comprising an amino acid sequence set forth in SEQ ID NO: 61 or having a variant with a substitution, deletion or insertion at a single site; and/or (c) a VH CDR3 comprising an amino acid sequence set forth in any one of SEQ ID NOs: 62-97 or 106 or having a variant with a substitution, deletion or insertion at a single site.

In some embodiments, provided is an antibody or an antigen-binding fragment specifically binding a spike protein and comprising:
(a) a VH CDR1 comprising an amino acid sequence set forth in SEQ ID NO: 60; (b) a VH CDR2 comprising an amino acid sequence set forth as SEQ ID NO: 61; and (c) a VH CDR3 comprising an amino acid sequence set forth in any one of SEQ ID NOs: 62-97 or 106.

In some embodiments, provided is an antibody or antigen-binding fragment specifically binding to a spike protein and comprising: (a) a VH CDR1 comprising an amino acid sequence set forth in SEQ ID NO: 60 or having a variant with a substitution, deletion or insertion at a single site; (b) a VH CDR2 comprising an amino acid sequence set forth in SEQ ID NO: 61 or having a variant with a substitution, deletion or insertion at a single site; (c) a VH CDR3 comprising an amino acid sequence set forth in any one of SEQ ID NOs: 62-97 or 106 or having a variant with a substitution, deletion or insertion at a single site; (d) a VL CDR1 comprising an amino acid sequence set forth in SEQ ID NO: 98 or having a variant with a substitution, deletion or insertion at a single site; (e) a VL CDR2 comprising an amino acid sequence set forth in SEQ ID NO: 99 or having a variant with a substitution, deletion or insertion at a single site; and/or (f) a VL CDR3 comprising an amino acid sequence set forth in SEQ ID NO: 100 or having a variant with a substitution, deletion or insertion at a single site. In some embodiments, a variant with a substitution is a variant with a conservative amino acid substitution.

In some embodiments, the antibody or the antigen-binding fragment at least comprises one, two, three, four, five, or all of the VH CDR1 set forth in SEQ ID NO: 60, the VH CDR2 set forth in SEQ ID NO: 61, the VH CDR3 set forth in any one of SEQ ID NOs: 62-97 or 106, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in any one of SEQ ID NOs: 62-97 or 106, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 62, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 63, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 64, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 65, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 66, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 67, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 68, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 69, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 70, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 71, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 72, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 73, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 74, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 75, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 76, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 77, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 78, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 79, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 80, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 81, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 82, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 83, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 84, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 85, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 86, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 87, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 88, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 89, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 90, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 91, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 92, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 93, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 94, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 95, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 96, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 97, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 106, the VL CDR1 set forth in SEQ ID NO: 98, the VL CDR2 set forth in SEQ ID NO: 99, and the VL CDR3 set forth in SEQ ID NO: 100.

In some embodiments, the heavy chain FR1 of the antibody or the antigen-binding fragment comprises a sequence set forth in SEQ ID NO: 101, a sequence having at least 90% identity to the sequence set forth in SEQ ID NO: 101, or an amino acid sequence having one or more conservative amino acid substitutions as compared to the sequence set forth in SEQ ID NO: 101; and/or the heavy chain FR2 of the antibody or the antigen-binding fragment comprises a sequence set forth in SEQ ID NO: 102, a sequence having at least 90% identity to the sequence set forth in SEQ ID NO: 102, or an amino acid sequence having one or more conservative amino acid substitutions as compared to the sequence set forth in SEQ ID NO: 102; and/or
the heavy chain FR3 of the antibody or the antigen-binding fragment comprises a sequence set forth in SEQ ID NO: 103, a sequence having at least 90% identity to the sequence set forth in SEQ ID NO: 103, or an amino acid sequence having one or more conservative amino acid substitutions as compared to the sequence set forth in SEQ ID NO: 103; and/or
the heavy chain FR4 of the antibody or the antigen-binding fragment comprises a sequence set forth in SEQ ID NO: 104, a sequence having at least 90% identity to the sequence set forth in SEQ ID NO: 104, or an amino acid sequence having one or more conservative amino acid substitutions as compared to the sequence set forth in SEQ ID NO: 104.

In some embodiments, the heavy chain FR1 comprises a sequence set forth in SEQ ID NO: 101, the heavy chain FR2 comprises a sequence set forth in SEQ ID NO: 102, the heavy chain FR3 comprises a sequence set forth in SEQ ID NO: 103, and the heavy chain FR4 comprises a sequence set forth in SEQ ID NO: 104.

In some embodiments, a heavy chain variable region of the antibody or the antigen-binding fragment comprises a sequence set forth in any one of SEQ ID NOs: 46-49 or 107, a sequence having at least 80% identity to the sequence set forth in any one of SEQ ID NOs: 46-49 or 107, or an amino acid sequence having one or more conservative amino acid substitutions as compared to the sequence set forth in any one of SEQ ID NOs: 46-49 or 107.

In some embodiments, a light chain variable region of the antibody or the antigen-binding fragment comprises a sequence set forth in SEQ ID NO: 50, a sequence having at least 80% identity to the sequence set forth in SEQ ID NO: 50, or an amino acid sequence having one or more conservative amino acid substitutions as compared to the sequence set forth in SEQ ID NO: 50.

In some embodiments, a heavy chain variable region of the antibody or the antigen-binding fragment comprises a sequence set forth in any one of SEQ ID NOs: 46-49 or 107, and/or a light chain variable region of the antibody or the antigen-binding fragment comprises a sequence set forth in SEQ ID NO: 50. In some embodiments, a heavy chain variable region of the antibody or the antigen-binding fragment comprises a sequence set forth in SEQ ID NO: 46, and the light chain variable region of the antibody or the antigen-binding fragment comprises a sequence set forth in SEQ ID NO: 50. In some embodiments, a heavy chain variable region of the antibody or the antigen-binding fragment comprises a sequence set forth in SEQ ID NO: 47, and the light chain variable region of the antibody or the antigen-binding fragment comprises a sequence set forth in SEQ ID NO: 50. In some embodiments, a heavy chain variable region of the antibody or the antigen-binding fragment comprises a sequence set forth in SEQ ID NO: 48, and the light chain variable region of the antibody or the antigen-binding fragment comprises a sequence set forth in SEQ ID NO: 50. In some embodiments, a heavy chain variable region of the antibody or the antigen-binding fragment comprises a sequence set forth in SEQ ID NO: 49, and the light chain variable region of the antibody or the antigen-binding fragment comprises a sequence set forth in SEQ ID NO: 50. In some embodiments, a heavy chain variable region of the antibody or the antigen-binding fragment comprises a sequence set forth in SEQ ID NO: 107, and the light chain variable region of the antibody or the antigen-binding fragment comprises a sequence set forth in SEQ ID NO: 50.

In some embodiments, the antibody or the antigen-binding fragment further comprises a heavy chain constant region, a light chain constant region, an Fc region, or a combination thereof. In some embodiments, the light chain constant region is a κ or λ chain constant region. In some embodiments, the antibody or the fragment thereof is of one of the isotypes IgG, IgM, IgA, IgE or IgD. In some embodiments, the isotype is IgG1, IgG2, IgG3 or IgG4. Without limitation, the antibody or the antigen-binding fragment is a chimeric antibody, a humanized antibody, or a fully human antibody. In a certain aspect, the antibody or the antigen-binding fragment is a humanized antibody.

In some embodiments, the antibody or the antigen-binding fragment is an isolated antibody or an antigen-binding fragment. In some embodiments, the antibody or the antigen-binding fragment is a scFV, a Fab, a F(ab)₂ or a IgG. In some embodiments, the antibody or the antigen-binding fragment is a monoclonal antibody.

In some embodiments, a heavy chain constant region of the antibody or the antigen-binding fragment comprises a sequence having an amino acid sequence set forth in SEQ ID NO: 51, a sequence having at least 80% identity to the sequence set forth in SEQ ID NO: 51, or an amino acid sequence having one or more conservative amino acid substitutions as compared to the sequence set forth in SEQ ID NO: 51; and/ or
a light chain constant region of the antibody or the antigen-binding fragment comprises a sequence having an amino acid sequence set forth in SEQ ID NO: 52, a sequence having at least 80% identity to the sequence set forth in SEQ ID NO: 52, or an amino acid sequence having one or more conservative amino acid substitutions as compared to the sequence set forth in SEQ ID NO: 52.

In some embodiments, a heavy chain constant region of the antibody or the antigen-binding fragment comprises a sequence having an amino acid sequence set forth in SEQ ID NO: 51, and/or a light chain constant region of the antibody or the antigen-binding fragment comprises a sequence having an amino acid sequence set forth in SEQ ID NO: 52. In some embodiments, a heavy chain constant region of the antibody or the antigen-binding fragment comprises a sequence having an amino acid sequence set forth in SEQ ID NO: 51, and the light chain constant region of the antibody or the antigen-binding fragment comprises a sequence having an amino acid sequence set forth in SEQ ID NO: 52.

In some embodiments, a heavy chain of the antibody comprises a sequence having an amino acid sequence set forth in any one of SEQ ID NOs: 53-56 or 108, a sequence having at least 80% identity to the sequence set forth in any one of SEQ ID NOs: 53-56 or 108, or an amino acid sequence having one or more conservative amino acid substitutions as compared to the sequence set forth in any one of SEQ ID NOs: 53-56 or 108; and/or
a light chain of the antibody comprises a sequence having an amino acid sequence set forth in SEQ ID NO: 57, a sequence having at least 80% identity to the sequence set forth in SEQ ID NO: 57, or an amino acid sequence having one or more conservative amino acid substitutions as compared to the sequence set forth in SEQ ID NO: 57.

In some embodiments, a heavy chain of the antibody comprises a sequence having an amino acid sequence set forth in any one of SEQ ID NOs: 53-56 or 108, and/or a light chain of the antibody comprises a sequence having an amino acid sequence set forth in SEQ ID NO: 57.

In some embodiments, a heavy chain of the antibody comprises a sequence having an amino acid sequence set forth in SEQ ID NO: 53, and a light chain of the antibody comprises a sequence having an amino acid sequence set forth in SEQ ID NO: 57. In some embodiments, a heavy chain of the antibody comprises a sequence having an amino acid sequence set forth in SEQ ID NO: 54, and a light chain of the antibody comprises a sequence having an amino acid sequence set forth in SEQ ID NO: 57. In some embodiments, a heavy chain of the antibody comprises a sequence having an amino acid sequence set forth in SEQ ID NO: 55, and a light chain of the antibody comprises a sequence having an amino acid sequence set forth in SEQ ID NO: 57. In some embodiments, a heavy chain of the antibody comprises a sequence having an amino acid sequence set forth in SEQ ID NO: 56, and a light chain of the antibody comprises a sequence having an amino acid sequence set forth in SEQ ID NO: 57. In some embodiments, a heavy chain of the antibody comprises a sequence having an amino acid sequence set forth in SEQ ID NO: 108, and a light chain of the antibody comprises a sequence having an amino acid sequence set forth in SEQ ID NO: 57.

In some embodiments, the antibody comprises 2 heavy chains with the same sequence and 2 light chains with the same sequence. In some embodiments, the antibody or the antigen-binding fragment can specifically bind to a spike protein and block binding of SARS-CoV or SARS-CoV-2 viral particles to cells, as well as mediate phagocytosis and clearance of the virus particles by immune cells. In some embodiments, the antibody or the antigen-binding fragment can specifically bind to the S1 subunit in a spike protein.

The present invention further provides a fusion protein. The antibody part or the antigen-binding fragment in the fusion protein has high affinity for the spike protein of SARS-CoV and SARS-CoV-2, and these antibodies can specifically bind to the spike protein; 6-HB interference polypeptides in the fusion protein can interfere with the formation of "six-helix bundle (6-HB)". The fusion protein of the present invention can prevent fusion of virus particles and cell membranes, and can mediate phagocytosis and clearance of the virus particles by immune cells. These fusion proteins may be used for treating or ameliorating SARS and COVID-19, and also for diagnosing SARS and COVID-19.

The virus particles first bind to angiotensin converting enzyme 2 (ACE2) on the surface of lung epithelial cells via the receptor binding domain (RBD) in the S1 subunit of spike protein (S protein) on the surface of the virus particles. After the virus binds to the receptor and is hydrolyzed by proteases, S2 subunit on the N-terminus of S protein is exposed and embedded into the plasma membrane or endosome membrane. The HR2 (heptad repeated 2) domain in the S2 subunit binds to the HR1 (heptad repeated 1) domain in the S2 subunit to form 6-HB fusion core, so that a viral coat is fused with a cell membrane, SARS-CoV or SARS-CoV-2 enters the cells and utilizes the cells to synthesize new virus particles; the new viral particles are released outside the cell and then infect surrounding normal cells in the same manner. The antibody in the fusion protein can block the binding of the spike protein to ACE2, and the 6-HB interference polypeptide moiety prevents the fusion of the viral coat with the cell membrane, thereby blocking the virus from entering the cells and playing a role of antiviral; the antibody moiety in the fusion protein may also mediate phagocytosis and clearance of the virus by immune cells.

In some embodiments, provided is a fusion protein comprising an antibody or an antigen-binding fragment, and a 6-HB interference polypeptide, wherein the antibody or the antigen-binding fragment specifically binds to a spike protein and comprises: (a) a VH CDR1 comprising an amino acid sequence set forth in SEQ ID NO: 1 or having a variant with a substitution, deletion or insertion at a single site; (b) a VH CDR2 comprising an amino acid sequence set forth in SEQ ID NO: 2 or having a variant with a substitution, deletion or insertion at a single site; and/or (c) a VH CDR3 comprising an amino acid sequence set forth in any one of SEQ ID Nos: 3-38 or 105 or having a variant with a substitution, deletion or insertion at a single site;
the C-terminus or N-terminus of the antibody or the antigen-binding fragment is linked to the 6-HB interference polypeptides via a linker. In some embodiments, the linker is a polypeptide comprising glycine and serine.

In some embodiments, provided is a fusion protein comprising an antibody or an antigen-binding fragment, and a 6-HB interference polypeptide, wherein the antibody or the antigen-binding fragment specifically binds to a spike protein and comprises: (a) a VH CDR1 comprising an amino acid sequence set forth in SEQ ID NO: 1 or having a variant with a substitution, deletion or insertion at a single site; (b) a VH CDR2 comprising an amino acid sequence set forth in SEQ ID NO: 2 or having a variant with a substitution, deletion or insertion at a single site; and (c) a VH CDR3 comprising an amino acid sequence set forth in any one of SEQ ID Nos: 3-38 or 105 or having a variant with a substitution, deletion or insertion at a single site;
the C-terminus or N-terminus of the antibody or the antigen-binding fragment is linked to the 6-HB interference polypeptides via a linker. In some embodiments, the linker is a polypeptide comprising glycine and serine.

In some embodiments, provided is a fusion protein comprising an antibody or an antigen-binding fragment, and a 6-HB interference polypeptide, wherein the antibody or the antigen-binding fragment specifically binds to a spike protein and comprises:
(a) a VH CDR1 comprising an amino acid sequence set forth in SEQ ID NO: 1; (b) a VH CDR2 comprising an amino acid sequence set forth as SEQ ID NO: 2; and (c) a VH CDR3 comprising an amino acid sequence set forth in any one of SEQ ID NOs: 3-38 or 105;
   the C-terminus or N-terminus of the antibody or the antigen-binding fragment is linked to the 6-HB interference polypeptides via a linker. In some embodiments, the linker is a polypeptide comprising glycine and serine.

In some embodiments, provided is a fusion protein comprising an antibody or an antigen-binding fragment, and a 6-HB interference polypeptide, wherein the antibody or the antigen-binding fragment specifically binds to a spike protein and comprises: (a) a VH CDR1 comprising an amino acid sequence set forth in SEQ ID NO: 1 or having a variant with a substitution, deletion or insertion at a single site; (b) a VH CDR2 comprising an amino acid sequence set forth in SEQ ID NO: 2 or having a variant with a substitution, deletion or insertion at a single site; (c) a VH CDR3 comprising an amino acid sequence set forth in any one of SEQ ID NOs: 3-38 or 105 or having a variant with a substitution, deletion or insertion at a single site; (d) a VL CDR1 comprising an amino acid sequence set forth in SEQ ID NO: 39 or having a variant with a substitution, deletion or insertion at a single site; (e) a VL CDR2 comprising an amino acid sequence set forth in SEQ ID NO: 40 or having a variant with a substitution, deletion or insertion at a single site; and/or (f) a VL CDR3 comprising an amino acid sequence set forth in SEQ ID NO: 41 or having a variant with a substitution, deletion or insertion at a single site;
the C-terminus or N-terminus of the antibody or the antigen-binding fragment is linked to the 6-HB interference polypeptides via a linker. In some embodiments, the linker is a polypeptide comprising glycine and serine.

In some embodiments, the fusion protein comprises an antibody or an antigen-binding fragment, and a 6-HB interference polypeptide, wherein the antibody or the antigen-binding fragment specifically binds to a spike protein and comprises: (a) a VH CDR1 comprising an amino acid sequence set forth in SEQ ID NO: 1 or having a variant with a substitution, deletion or insertion at a single site; (b) a VH CDR2 comprising an amino acid sequence set forth in SEQ ID NO: 2 or having a variant with a substitution, deletion or insertion at a single site; (c) a VH CDR3 comprising an amino acid sequence set forth in any one of SEQ ID NOs: 3-38 or 105 or having a variant with a substitution, deletion or insertion at a single site; (d) a VL CDR1 comprising an amino acid sequence set forth in SEQ ID NO: 39 or having a variant with a substitution, deletion or insertion at a single site; (e) a VL CDR2 comprising an amino acid sequence set forth in SEQ ID NO: 40 or having a variant with a substitution, deletion or insertion at a single site; and/or (f) a VL CDR3 comprising an amino acid sequence set forth in SEQ ID NO: 41 or having a variant with a substitution, deletion or insertion at a single site;
the C-terminus or N-terminus of the antibody or the antigen-binding fragment is linked to the 6-HB interference polypeptides via a linker. In some embodiments, the linker is a polypeptide comprising glycine and serine. In some embodiments, a variant with a substitution is a variant with a conservative amino acid substitution.

In some embodiments, a sequence of the linker is (GₘS)ₙ, and each m is independently 2, 3, 4 or 5, and n is 1, 2, 3, 4 or 5. In some embodiments, a sequence of the linker is (GGGGS)ₙ, and the n is 1, 2, 3, 4 or 5. In some embodiments, a sequence of the linker is GGGGS. In some embodiments, a sequence of the linker is (GGGGS)₂. In some embodiments, a sequence of the linker is (GGGGS)₃. In some embodiments, a sequence of the linker is (GGGGS)₄ set forth in SEQ ID NO: 59. In some embodiments, a sequence of the linker is (GGGGS)₅.

In some embodiments, the 6-HB interference polypeptide comprises a sequence set forth in SEQ ID NO: 58, a sequence having at least 90% identity to the sequence set forth in SEQ ID NO: 58, or an amino acid sequence having one or more conservative amino acid substitutions as compared to the sequence set forth in SEQ ID NO: 58.

In some embodiments, the fusion protein comprises an antibody or antigen-binding fragment, and a 6-HB interference polypeptide, and the fusion protein comprises the following features:
the antibody or the antigen-binding fragment at least comprises one, two, three, four, five, or all of the VH CDR1 set forth in SEQ ID NO: 1, the VH CDR2 set forth in SEQ ID NO: 2, the VH CDR3 set forth in any one of SEQ ID NOs: 3-38 or 105, the VL CDR1 set forth in SEQ ID NO: 39, the VL CDR2 set forth in SEQ ID NO: 40, and the VL CDR3 set forth in SEQ ID NO: 41; and/or
the C-terminus of the antibody or the antigen-binding fragment is linked to the 6-HB interference polypeptides via a linker, and the C-terminus of the antibody or the antigen-binding fragment is a C-terminus of the heavy chain or a C-terminus of the light chain of the antibody or the antigen-binding fragment; and/or
a sequence of the linker is (GGGGS)ₙ, and the n is 1, 2, 3, 4 or 5; and/or
the 6-HB interference polypeptide comprises a sequence set forth in SEQ ID NO: 58, a sequence having at least 90% identity to the sequence set forth in SEQ ID NO: 58, or an amino acid sequence having one or more conservative amino acid substitutions as compared to the sequence set forth in SEQ ID NO: 58.

In some embodiments, the fusion protein comprises an antibody or antigen-binding fragment, and a 6-HB interference polypeptide, and the fusion protein comprises the following features:
the antibody or the antigen-binding fragment at least comprises the VH CDR1 set forth in SEQ ID NO: 1, the VH CDR2 set forth in SEQ ID NO: 2, the VH CDR3 set forth in any one of SEQ ID NOs: 3-38 or 105, the VL CDR1 set forth in SEQ ID NO: 39, the VL CDR2 set forth in SEQ ID NO: 40, and the VL CDR3 set forth in SEQ ID NO: 41; and/or
the C-terminus of the antibody or the antigen-binding fragment is linked to the 6-HB interference polypeptides via a linker, and the C-terminus of the antibody or the antigen-binding fragment is a C-terminus of the heavy chain or a C-terminus of the light chain of the antibody or the antigen-binding fragment; and/or
a sequence of the linker is (GGGGS)ₙ, and the n is 1, 2, 3, 4 or 5; and/or
the 6-HB interference polypeptide comprises a sequence set forth in SEQ ID NO: 58, a sequence having at least 90% identity to the sequence set forth in SEQ ID NO: 58, or an amino acid sequence having one or more conservative amino acid substitutions as compared to the sequence set forth in SEQ ID NO: 58.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 3, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 4, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 5, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 6, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 7, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 8, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 9, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 10, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 11, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 12, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 13, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 14, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 15, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 16, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 17, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 18, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 19, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 20, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 21, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 22, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 23, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 24, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 25, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 26, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 27, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 28, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 29, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 30, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 31, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 32, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 33, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 34, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 35, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 36, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 37, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 38, the VL CDR1 set forth in SEQ ID NO: 39, the VL CDR2 set forth in SEQ ID NO: 40, and the VL CDR3 set forth in SEQ ID NO: 41.

In some embodiments, the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 105, the VL CDR1 set forth in SEQ ID NO: 39, the VL CDR2 set forth in SEQ ID NO: 40, and the VL CDR3 set forth in SEQ ID NO: 41.

In some embodiments, framework regions of a heavy chain variable region of the antibody or the antigen-binding fragment comprise a heavy chain FR1, a heavy chain FR2, a heavy chain FR3 and a heavy chain FR4, and the heavy chain FR1 of the antibody or the antigen-binding fragment comprises a sequence set forth in SEQ ID NO: 42, a sequence having at least 90% identity to the sequence set forth in SEQ ID NO: 42, or an amino acid sequence having one or more conservative amino acid substitutions as compared to the sequence set forth in SEQ ID NO: 42; and/or
the heavy chain FR2 of the antibody or the antigen-binding fragment comprises a sequence set forth in SEQ ID NO: 43, a sequence having at least 90% identity to the sequence set forth in SEQ ID NO: 43, or an amino acid sequence having one or more conservative amino acid substitutions as compared to the sequence set forth in SEQ ID NO: 43; and/or
the heavy chain FR3 of the antibody or the antigen-binding fragment comprises a sequence set forth in SEQ ID NO: 44, a sequence having at least 90% identity to the sequence set forth in SEQ ID NO: 44, or an amino acid sequence having one or more conservative amino acid substitutions as compared to the sequence set forth in SEQ ID NO: 44; and/or
the heavy chain FR4 of the antibody or the antigen-binding fragment comprises a sequence set forth in SEQ ID NO: 45, a sequence having at least 90% identity to the sequence set forth in SEQ ID NO: 45, or an amino acid sequence having one or more conservative amino acid substitutions as compared to the sequence set forth in SEQ ID NO: 45.

In some embodiments, the heavy chain FR1 comprises a sequence set forth in SEQ ID NO: 42, a sequence having at least 90% identity to the sequence set forth in SEQ ID NO: 42, or an amino acid sequence having one or more conservative amino acid substitutions as compared to the sequence set forth in SEQ ID NO: 42; the heavy chain FR2 comprises a sequence set forth in SEQ ID NO: 43, a sequence having at least 90% identity to the sequence set forth in SEQ ID NO: 43, or an amino acid sequence having one or more conservative amino acid substitutions as compared to the sequence set forth in SEQ ID NO: 43; the heavy chain FR3 comprises a sequence set forth in SEQ ID NO: 44, a sequence having at least 90% identity to the sequence set forth in SEQ ID NO: 44, or an amino acid sequence having one or more conservative amino acid substitutions as compared to the sequence set forth in SEQ ID NO: 44; the heavy chain FR4 comprises a sequence set forth in SEQ ID NO: 45, a sequence having at least 90% identity to the sequence set forth in SEQ ID NO: 45, or an amino acid sequence having one or more conservative amino acid substitutions as compared to the sequence set forth in SEQ ID NO: 45.

In some embodiments, the heavy chain FR1 comprises a sequence set forth in SEQ ID NO: 42, the heavy chain FR2 comprises a sequence set forth in SEQ ID NO: 43, the heavy chain FR3 comprises a sequence set forth in SEQ ID NO: 44, and the heavy chain FR4 comprises a sequence set forth in SEQ ID NO: 45.

In some embodiments, provided is a fusion protein comprising an antibody or an antigen-binding fragment, and a 6-HB interference polypeptide, wherein the antibody or the antigen-binding fragment specifically binds to a spike protein and comprises: (a) a VH CDR1 comprising an amino acid sequence set forth in SEQ ID NO: 60 or having a variant with a substitution, deletion or insertion at a single site; (b) a VH CDR2 comprising an amino acid sequence set forth in SEQ ID NO: 61 or having a variant with a substitution, deletion or insertion at a single site; and/or (c) a VH CDR3 comprising an amino acid sequence set forth in any one of SEQ ID Nos: 62-97 or 106 or having a variant with a substitution, deletion or insertion at a single site;
the C-terminus or N-terminus of the antibody or the antigen-binding fragment is linked to the 6-HB interference polypeptides via a linker. In some embodiments, the linker is a polypeptide comprising glycine and serine.

In some embodiments, provided is a fusion protein comprising an antibody or an antigen-binding fragment, and a 6-HB interference polypeptide, wherein the antibody or the antigen-binding fragment specifically binds to a spike protein and comprises:
(a) a VH CDR1 comprising an amino acid sequence set forth in SEQ ID NO: 60; (b) a VH CDR2 comprising an amino acid sequence set forth as SEQ ID NO: 61; and (c) a VH CDR3 comprising an amino acid sequence set forth in any one of SEQ ID NOs: 62-97 or 106;
   the C-terminus or N-terminus of the antibody or the antigen-binding fragment is linked to the 6-HB interference polypeptides via a linker. In some embodiments, the linker is a polypeptide comprising glycine and serine.

In some embodiments, provided is a fusion protein comprising an antibody or an antigen-binding fragment, and a 6-HB interference polypeptide, wherein the antibody or the antigen-binding fragment specifically binds to a spike protein and comprises: (a) a VH CDR1 comprising an amino acid sequence set forth in SEQ ID NO: 60 or having a variant with a substitution, deletion or insertion at a single site; (b) a VH CDR2 comprising an amino acid sequence set forth in SEQ ID NO: 61 or having a variant with a substitution, deletion or insertion at a single site; (c) a VH CDR3 comprising an amino acid sequence set forth in any one of SEQ ID NOs: 62-97 or 106 or having a variant with a substitution, deletion or insertion at a single site; (d) a VL CDR1 comprising an amino acid sequence set forth in SEQ ID NO: 98 or having a variant with a substitution, deletion or insertion at a single site; (e) a VL CDR2 comprising an amino acid sequence set forth in SEQ ID NO: 99 or having a variant with a substitution, deletion or insertion at a single site; and/or (f) a VL CDR3 comprising an amino acid sequence set forth in SEQ ID NO: 100 or having a variant with a substitution, deletion or insertion at a single site;
the C-terminus or N-terminus of the antibody or the antigen-binding fragment is linked to the 6-HB interference polypeptides via a linker. In some embodiments, the linker is a polypeptide comprising glycine and serine.

In some embodiments, a sequence of the linker is (GₘS)ₙ, and each m is independently 2, 3, 4 or 5, and n is 1, 2, 3, 4 or 5. In some embodiments, a sequence of the linker is (GGGGS)ₙ, and the n is 1, 2, 3, 4 or 5. In some embodiments, a sequence of the linker is GGGGS. In some embodiments, a sequence of the linker is (GGGGS)₂. In some embodiments, a sequence of the linker is (GGGGS)₃. In some embodiments, a sequence of the linker is (GGGGS)₄ set forth in SEQ ID NO: 59. In some embodiments, a sequence of the linker is (GGGGS)₅.

In some embodiments, the 6-HB interference polypeptide comprises a sequence set forth in SEQ ID NO: 58, a sequence having at least 90% identity to the sequence set forth in SEQ ID NO: 58, or an amino acid sequence having one or more conservative amino acid substitutions as compared to the sequence set forth in SEQ ID NO: 58.

In some embodiments, the fusion protein comprises an antibody or antigen-binding fragment, and a 6-HB interference polypeptide, and the fusion protein comprises the following features:
the antibody or the antigen-binding fragment at least comprises one, two, three, four, five, or all of the VH CDR1 set forth in SEQ ID NO: 60, the VH CDR2 set forth in SEQ ID NO: 61, the VH CDR3 set forth in any one of SEQ ID NOs: 62-97 or 106, the VL CDR1 set forth in SEQ ID NO: 98, the VL CDR2 set forth in SEQ ID NO: 99, and the VL CDR3 set forth in SEQ ID NO: 100; and/or
the C-terminus of the antibody or the antigen-binding fragment is linked to the 6-HB interference polypeptides via a linker, and the C-terminus of the antibody or the antigen-binding fragment is a C-terminus of the heavy chain or a C-terminus of the light chain of the antibody or the antigen-binding fragment; and/or
a sequence of the linker is (GGGGS)ₙ, and the n is 1, 2, 3, 4 or 5; and/or
the 6-HB interference polypeptide comprises a sequence set forth in SEQ ID NO: 58, a sequence having at least 90% identity to the sequence set forth in SEQ ID NO: 58, or an amino acid sequence having one or more conservative amino acid substitutions as compared to the sequence set forth in SEQ ID NO: 58.

In some embodiments, the fusion protein comprises an antibody or antigen-binding fragment, and a 6-HB interference polypeptide, and the fusion protein comprises the following features:
the antibody or the antigen-binding fragment at least comprises the VH CDR1 set forth in SEQ ID NO: 60, the VH CDR2 set forth in SEQ ID NO: 61, the VH CDR3 set forth in any one of SEQ ID NOs: 62-97 or 106, the VL CDR1 set forth in SEQ ID NO: 98, the VL CDR2 set forth in SEQ ID NO: 99, and the VL CDR3 set forth in SEQ ID NO: 100; and/or
the C-terminus of the antibody or the antigen-binding fragment is linked to the 6-HB interference polypeptides via a linker, and the C-terminus of the antibody or the antigen-binding fragment is a C-terminus of the heavy chain or a C-terminus of the light chain of the antibody or the antigen-binding fragment; and/or
a sequence of the linker is (GGGGS)ₙ, and the n is 1, 2, 3, 4 or 5; and/or
the 6-HB interference polypeptide comprises a sequence set forth in SEQ ID NO: 58, a sequence having at least 90% identity to the sequence set forth in SEQ ID NO: 58, or an amino acid sequence having one or more conservative amino acid substitutions as compared to the sequence set forth in SEQ ID NO: 58.

In some embodiments, the antibody or the antigen-binding fragment at least comprises the VH CDR1 set forth in SEQ ID NO: 60, the VH CDR2 set forth in SEQ ID NO: 61, the VH CDR3 set forth in any one of SEQ ID NOs: 62-97 or 106, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 62, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 63, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 64, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 65, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 66, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 67, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 68, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 69, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 70, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 71, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 72, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 73, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 74, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 75, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 76, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 77, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 78, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 79, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 80, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 81, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 82, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 83, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 84, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 85, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 86, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 87, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 88, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 89, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 90, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 91, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 92, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 93, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 94, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 95, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 96, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 97, the VL CDR1 set forth in SEQ ID NO: 98, the VL CDR2 set forth in SEQ ID NO: 99, and the VL CDR3 set forth in SEQ ID NO: 100.

In some embodiments, the antibody or the antigen-binding fragment at least comprises the VH CDR1 set forth in SEQ ID NO: 60, the VH CDR2 set forth in SEQ ID NO: 61, the VH CDR3 set forth in SEQ ID NO: 106, the VL CDR1 set forth in SEQ ID NO: 98, the VL CDR2 set forth in SEQ ID NO: 99, and the VL CDR3 set forth in SEQ ID NO: 100.

In some embodiments, the heavy chain FR1 of the antibody or the antigen-binding fragment comprises a sequence set forth in SEQ ID NO: 101, a sequence having at least 90% identity to the sequence set forth in SEQ ID NO: 101, or an amino acid sequence having one or more conservative amino acid substitutions as compared to the sequence set forth in SEQ ID NO: 101; and/or
the heavy chain FR2 of the antibody or the antigen-binding fragment comprises a sequence set forth in SEQ ID NO: 102, a sequence having at least 90% identity to the sequence set forth in SEQ ID NO: 102, or an amino acid sequence having one or more conservative amino acid substitutions as compared to the sequence set forth in SEQ ID NO: 102; and/or
the heavy chain FR3 of the antibody or the antigen-binding fragment comprises a sequence set forth in SEQ ID NO: 103, a sequence having at least 90% identity to the sequence set forth in SEQ ID NO: 103, or an amino acid sequence having one or more conservative amino acid substitutions as compared to the sequence set forth in SEQ ID NO: 103; and/or
the heavy chain FR4 of the antibody or the antigen-binding fragment comprises a sequence set forth in SEQ ID NO: 104, a sequence having at least 90% identity to the sequence set forth in SEQ ID NO: 104, or an amino acid sequence having one or more conservative amino acid substitutions as compared to the sequence set forth in SEQ ID NO: 104.

In some embodiments, the heavy chain FR1 comprises a sequence set forth in SEQ ID NO: 101, the heavy chain FR2 comprises a sequence set forth in SEQ ID NO: 102, the heavy chain FR3 comprises a sequence set forth in SEQ ID NO: 103, and the heavy chain FR4 comprises a sequence set forth in SEQ ID NO: 104.

In some embodiments, a heavy chain variable region of the antibody or the antigen-binding fragment comprises a sequence set forth in any one of SEQ ID NOs: 46-49 or 107, and/or the light chain variable region of the antibody or the antigen-binding fragment comprises a sequence set forth in SEQ ID NO: 50.

In some embodiments, a heavy chain variable region of the antibody or the antigen-binding fragment comprises a sequence set forth in SEQ ID NO: 46, and the light chain variable region of the antibody or the antigen-binding fragment comprises a sequence set forth in SEQ ID NO: 50. In some embodiments, a heavy chain variable region of the antibody or the antigen-binding fragment comprises a sequence set forth in SEQ ID NO: 47, and the light chain variable region of the antibody or the antigen-binding fragment comprises a sequence set forth in SEQ ID NO: 50. In some embodiments, a heavy chain variable region of the antibody or the antigen-binding fragment comprises a sequence set forth in SEQ ID NO: 48, and the light chain variable region of the antibody or the antigen-binding fragment comprises a sequence set forth in SEQ ID NO: 50. In some embodiments, a heavy chain variable region of the antibody or the antigen-binding fragment comprises a sequence set forth in SEQ ID NO: 49, and the light chain variable region of the antibody or the antigen-binding fragment comprises a sequence set forth in SEQ ID NO: 50. In some embodiments, a heavy chain variable region of the antibody or the antigen-binding fragment comprises a sequence set forth in SEQ ID NO: 107, and the light chain variable region of the antibody or the antigen-binding fragment comprises a sequence set forth in SEQ ID NO: 50.

In some embodiments, the antibody or the antigen-binding fragment further comprises a heavy chain constant region, a light chain constant region, an Fc region, or a combination thereof. In some embodiments, the light chain constant region is a κ or λ chain constant region. In some embodiments, the antibody or the fragment thereof is of one of the isotypes IgG, IgM, IgA, IgE or IgD. In some embodiments, the isotype is IgG1, IgG2, IgG3 or IgG4. Without limitation, the antibody or the antigen-binding fragment is a chimeric antibody, a humanized antibody, or a fully human antibody. In a certain aspect, the antibody or the antigen-binding fragment is a humanized antibody.

In some embodiments, the antibody or the antigen-binding fragment is an isolated antibody or an antigen-binding fragment. In some embodiments, the antibody or the antigen-binding fragment is a scFV, a Fab, a F(ab)₂ or a IgG. In some embodiments, the antibody or the antigen-binding fragment is a monoclonal antibody.

In some embodiments, a heavy chain constant region of the antibody or the antigen-binding fragment comprises a sequence having an amino acid sequence set forth in SEQ ID NO: 51 or a sequence of amino acids at positions 1-328 of SEQ ID NO: 51, or a sequence having at least 80% identity to the sequence set forth in SEQ ID NO: 51 or the sequence of amino acids at positions 1-328 of SEQ ID NO: 51, or an amino acid sequence having one or more conservative amino acid substitutions as compared to the sequence set forth in SEQ ID NO: 51 or the sequence of amino acids at positions 1-328 of SEQ ID NO: 51; and/or
a light chain constant region of the antibody or the antigen-binding fragment comprises a sequence having an amino acid sequence set forth in SEQ ID NO: 52, a sequence having at least 80% identity to the sequence set forth in SEQ ID NO: 52, or an amino acid sequence having one or more conservative amino acid substitutions as compared to the sequence set forth in SEQ ID NO: 52.

In some embodiments, a heavy chain constant region of the antibody or the antigen-binding fragment comprises a sequence having an amino acid sequence set forth in SEQ ID NO: 51, and the light chain constant region of the antibody or the antigen-binding fragment comprises a sequence having an amino acid sequence set forth in SEQ ID NO: 52.

In some embodiments, a heavy chain constant region of the antibody or the antigen-binding fragment comprises a sequence having an amino acid sequence set forth in a sequence of amino acids at positions 1-328 of SEQ ID NO: 51, and a light chain constant region of the antibody or the antigen-binding fragment comprises a sequence having an amino acid sequence set forth in SEQ ID NO: 52.

In some embodiments, provided is a fusion protein comprising the following features:
a heavy chain of the antibody comprises a sequence having an amino acid sequence set forth in any one of SEQ ID NOs: 53-56 and 108 or a sequence of amino acids at positions 1-450 of any one of SEQ ID NOs: 53-56 and 108, or a sequence having at least 80% identity to the sequence set forth in any one of SEQ ID NOs: 53-56 and 108 or the sequence of amino acids at positions 1-450 of any one of SEQ ID NOs: 53-56 and 108, or an amino acid sequence having one or more conservative amino acid substitutions as compared to the sequence set forth in any one of SEQ ID NOs: 53-56 and 108 or the sequence of amino acids at positions 1-450 of any one of SEQ ID NOs: 53-56 and 108; and/or
a light chain of the antibody comprises a sequence having an amino acid sequence set forth in SEQ ID NO: 57, a sequence having at least 80% identity to the sequence set forth in SEQ ID NO: 57, or an amino acid sequence having one or more conservative amino acid substitutions as compared to the sequence set forth in SEQ ID NO: 57; and/or
a C-terminus of the heavy chain or a C-terminus of the light chain of the antibody is covalently linked, via a linker set forth in SEQ ID NO: 59, to the 6-HB interference polypeptide, wherein the 6-HB interference polypeptide comprises a sequence set forth in SEQ ID NO: 58, a sequence having at least 90% or at least 95% identity to the sequence set forth in SEQ ID NO: 58, or an amino acid sequence having one or more conservative amino acid substitutions as compared to the sequence set forth in SEQ ID NO: 58.

In some embodiments, the fusion protein comprises an antibody and a 6-HB interference polypeptide linked via a linker, a heavy chain of the antibody comprises a sequence having an amino acid sequence set forth in SEQ ID NO: 53, and a light chain of the antibody comprises a sequence having an amino acid sequence set forth in SEQ ID NO: 57; a C-terminus of the heavy chain (i.e., CH3 terminus) or a C-terminus of the light chain (i.e., CL terminus) of the antibody is covalently linked, via a linker set forth in SEQ ID NO: 59, to the 6-HB interference polypeptide, wherein the 6-HB interference polypeptide comprises a sequence set forth in SEQ ID NO: 58.

In some embodiments, the fusion protein comprises an antibody and a 6-HB interference polypeptide linked via a linker, a heavy chain of the antibody comprises a sequence having an amino acid sequence of amino acids at positions 1-450 of SEQ ID NO: 53, and a light chain of the antibody comprises a sequence having an amino acid sequence set forth in SEQ ID NO: 57; a C-terminus of the heavy chain (i.e., CH3 terminus) or a C-terminus of the light chain (i.e., CL terminus) of the antibody is covalently linked, via a linker set forth in SEQ ID NO: 59, to the 6-HB interference polypeptide, wherein the 6-HB interference polypeptide comprises a sequence set forth in SEQ ID NO: 58.

In some embodiments, the fusion protein comprises an antibody and a 6-HB interference polypeptide linked via a linker, a heavy chain of the antibody comprises a sequence having an amino acid sequence set forth in SEQ ID NO: 54, and a light chain of the antibody comprises a sequence having an amino acid sequence set forth in SEQ ID NO: 57; a C-terminus of the heavy chain (i.e., CH3 terminus) or a C-terminus of the light chain (i.e., CL terminus) of the antibody is covalently linked, via a linker set forth in SEQ ID NO: 59, to the 6-HB interference polypeptide, wherein the 6-HB interference polypeptide comprises a sequence set forth in SEQ ID NO: 58.

In some embodiments, the fusion protein comprises an antibody and a 6-HB interference polypeptide linked via a linker, a heavy chain of the antibody comprises a sequence having an amino acid sequence of amino acids at positions 1-450 of SEQ ID NO: 54, and a light chain of the antibody comprises a sequence having an amino acid sequence set forth in SEQ ID NO: 57; a C-terminus of the heavy chain (i.e., CH3 terminus) or a C-terminus of the light chain (i.e., CL terminus) of the antibody is covalently linked, via a linker set forth in SEQ ID NO: 59, to the 6-HB interference polypeptide, wherein the 6-HB interference polypeptide comprises a sequence set forth in SEQ ID NO: 58.

In some embodiments, the fusion protein comprises an antibody and a 6-HB interference polypeptide linked via a linker, a heavy chain of the antibody comprises a sequence having an amino acid sequence set forth in SEQ ID NO: 55, and a light chain of the antibody comprises a sequence having an amino acid sequence set forth in SEQ ID NO: 57; a C-terminus of the heavy chain (i.e., CH3 terminus) or a C-terminus of the light chain (i.e., CL terminus) of the antibody is covalently linked, via a linker set forth in SEQ ID NO: 59, to the 6-HB interference polypeptide, wherein the 6-HB interference polypeptide comprises a sequence set forth in SEQ ID NO: 58.

In some embodiments, the fusion protein comprises an antibody and a 6-HB interference polypeptide linked via a linker, a heavy chain of the antibody comprises a sequence having an amino acid sequence of amino acids at positions 1-450 of SEQ ID NO: 55, and a light chain of the antibody comprises a sequence having an amino acid sequence set forth in SEQ ID NO: 57; a C-terminus of the heavy chain (i.e., CH3 terminus) or a C-terminus of the light chain (i.e., CL terminus) of the antibody is covalently linked, via a linker set forth in SEQ ID NO: 59, to the 6-HB interference polypeptide, wherein the 6-HB interference polypeptide comprises a sequence set forth in SEQ ID NO: 58.

In some embodiments, the fusion protein comprises an antibody and a 6-HB interference polypeptide linked via a linker, a heavy chain of the antibody comprises a sequence having an amino acid sequence set forth in SEQ ID NO: 56, and a light chain of the antibody comprises a sequence having an amino acid sequence set forth in SEQ ID NO: 57; a C-terminus of the heavy chain (i.e., CH3 terminus) or a C-terminus of the light chain (i.e., CL terminus) of the antibody is covalently linked, via a linker set forth in SEQ ID NO: 59, to the 6-HB interference polypeptide, wherein the 6-HB interference polypeptide comprises a sequence set forth in SEQ ID NO: 58.

In some embodiments, the fusion protein comprises an antibody and a 6-HB interference polypeptide linked via a linker, a heavy chain of the antibody comprises a sequence having an amino acid sequence of amino acids at positions 1-450 of SEQ ID NO: 56, and a light chain of the antibody comprises a sequence having an amino acid sequence set forth in SEQ ID NO: 57; a C-terminus of the heavy chain (i.e., CH3 terminus) or a C-terminus of the light chain (i.e., CL terminus) of the antibody is covalently linked, via a linker set forth in SEQ ID NO: 59, to the 6-HB interference polypeptide, wherein the 6-HB interference polypeptide comprises a sequence set forth in SEQ ID NO: 58.

In some embodiments, the fusion protein comprises an antibody and a 6-HB interference polypeptide linked via a linker, a heavy chain of the antibody comprises a sequence having an amino acid sequence set forth in SEQ ID NO: 108, and a light chain of the antibody comprises a sequence having an amino acid sequence set forth in SEQ ID NO: 57; a C-terminus of the heavy chain (i.e., CH3 terminus) or a C-terminus of the light chain (i.e., CL terminus) of the antibody is covalently linked, via a linker set forth in SEQ ID NO: 59, to the 6-HB interference polypeptide, wherein the 6-HB interference polypeptide comprises a sequence set forth in SEQ ID NO: 58.

In some embodiments, the fusion protein comprises an antibody and a 6-HB interference polypeptide linked via a linker, a heavy chain of the antibody comprises a sequence having an amino acid sequence of amino acids at positions 1-450 of SEQ ID NO: 108, and a light chain of the antibody comprises a sequence having an amino acid sequence set forth in SEQ ID NO: 57; a C-terminus of the heavy chain (i.e., CH3 terminus) or a C-terminus of the light chain (i.e., CL terminus) of the antibody is covalently linked, via a linker set forth in SEQ ID NO: 59, to the 6-HB interference polypeptide, wherein the 6-HB interference polypeptide comprises a sequence set forth in SEQ ID NO: 58.

In some embodiments, the fusion protein comprises 2 heavy chains (or heavy chain fragments) with the same sequence and 2 light chains with the same sequence, and a C-terminus of each heavy chain is covalently linked, via a linker, to the 6-HB interference polypeptide. In some embodiments, the fusion protein comprises 2 heavy chains with the same sequence and 2 light chains with the same sequence, and a C-terminus of the light chain is covalently linked, via a linker, to the 6-HB interference polypeptide.

In some embodiments, the antibody moiety or the antigen-binding fragment in the fusion protein can specifically bind to the spike protein, and prevent binding of SARS-CoV or SARS-CoV-2 viral particles to cells, as well as mediate phagocytosis and clearance of the virus particles by immune cells. In some embodiments, the antibody or the antigen-binding fragment in the fusion protein can specifically bind to the S1 subunit in a spike protein.

The present invention further provides a nucleic acid molecule encoding the antibody, the antigen-binding fragment thereof or the fusion protein thereof. In some embodiments, the nucleic acid molecule is an isolated nucleic acid molecule.

The present invention further provides a vector comprising the nucleic acid molecule. In some embodiments, the vector is an isolated vector.

The present invention further provides a host cell comprising the nucleic acid molecule. In some embodiments, the host cell is an isolated host cell.

The present invention further provides a host cell comprising the vector. In some embodiments, the host cell is an isolated host cell. In some embodiments, the host cells are CHO cells, 293 cells, Cos1 cells, Cos7 cells, CV1 cells and murine L cells.

The present invention further provides a pharmaceutical composition comprising the antibody, the antigen-binding fragment thereof or the fusion protein thereof, and a pharmaceutically acceptable carrier.

The present invention further provides a treatment method and use. In some embodiments, provided is a method for treating or ameliorating SARS or COVID-19, wherein the method comprises administering to a patient an effective dose of the antibody, the antigen-binding fragment thereof or the fusion protein. In some embodiments, provided is use of the antibody, the antigen-binding fragment thereof or the fusion protein in the treatment or amelioration of SARS or COVID-19. In some embodiments, provided is use of the antibody, the antigen-binding fragment thereof or the fusion protein in the manufacture of a medicament for treating or ameliorating SARS or COVID-19.

The present invention further provides a diagnostic method and use. In some embodiments, provided is a method for detecting SARS-CoV or SARS-CoV-2 expression in a sample, wherein the method comprises contacting the sample with the antibody, the antigen-binding fragment thereof or the fusion protein such that the antibody, the antigen-binding fragment thereof, or an antibody or an antigen-binding fragment of the fusion protein binds to a spike protein, and detecting the binding, i.e., an amount of the spike protein in the sample. In some embodiments, provided is use in the manufacture of a kit for diagnosing SARS or COVID-19. In some embodiments, provided is a diagnostic kit comprising the antibody, the antigen-binding fragment thereof or the fusion protein.

The present invention provides an antibody, an antigen-binding fragment thereof or a fusion protein for the treatment of coronavirus, wherein a 6-HB interference polypeptide in the fusion protein work synergistically with the antibody moiety or the antigen-binding fragment to prevent fusion of SARS-CoV or SARS-CoV-2 viral particles with cells, and mediates phagocytosis and clearance of the virus particles by immune cells, thus treating SARS or COVID-19; the antibody, the antigen-binding fragments thereof, or the antibody or the antigen-binding fragment in the fusion protein of the present invention can also be used for diagnostic detection of whether a patient is infected with SARS-CoV or SARS-CoV-2.

### BRIEF DESCRIPTION OF THE DRAWINGS

FIG. 1 is a graph of the inhibition of binding of SARS-CoV-2 to ACE2 by a part of an anti-spike protein antibody of the present invention in an ELISA assay, wherein the abscissa represents concentration and the ordinate represents OD value; wherein 1 represents antibody 1, 7 represents antibody 7, 8 represents antibody 8, 9 represents antibody 9, 12 represents antibody 12, 18 represents antibody 18, 19 represents antibody 19, 20 represents antibody 20, 21 represents antibody 21, and 22 represents antibody 22.
FIG. 2 shows binding of the antibody and fusion protein A to Spike S 1 RBD; wherein, the Spike S 1 RBD in FIG. 2A is of wild-type, and Spike S 1 RBD in FIG. 2B comprises a wild-type Spike S 1 RBD and a variant thereof.
FIG. 3 shows that fusion protein A blocks binding of the Spike S 1 RBD to ACE2.
FIG. 4 shows binding of fusion protein A to CHO cells over-expressing spike protein; wherein the ordinate represents average fluorescence intensity and the abscissa represents concentration.
FIG. 5 shows that fusion protein A inhibits infection of pseudovirus into Vero cells; wherein the ordinate represents inhibition rate and the abscissa represents concentration, and sACE2 represents soluble ACE2 as control.

### Terms

Unless otherwise specified, each of the following terms shall have the meaning described below.

### Definitions

It should be noted that the term "an" entity refers to one or more of the entities. For example, "an antibody" should be interpreted as one or more antibodies. As such, the terms "a" (or "an"), "one or more" and "at least one" can be used interchangeably herein.

The term "polypeptide" is intended to encompass both the singular form "polypeptide" and the plural form "polypeptides", and refers to a molecule consisting of amino acid monomer linearly linked by amide bonds (or peptide bonds). The term "polypeptide" refers to any single chain or multiple chains of two or more amino acids and does not refer to a particular length of the product. Thus, included within the definition of "polypeptide" are peptides, dipeptides, tripeptides, oligopeptides, "proteins", "amino acid chains" or any other term used to refer to chains of two or more amino acids, and the term "polypeptide" may be used in place of, or interchangeably with, any of the above terms. The term "polypeptide" is also intended to refer to a product of post-expression polypeptide modification, including but not limited to glycosylation, acetylation, phosphorylation, amidation, derivatization by known protecting/blocking groups, proteolytic cleavage or non-naturally occurring amino acid modification. The polypeptide may be derived from a natural biological source or produced by recombinant techniques, but it is not necessarily translated from a specified nucleic acid sequence, and it may be produced in any manner, including chemical synthesis.

"Amino acid" refers to an organic compound containing both an amino group and a carboxyl group, such as an α-amino acid that can be encoded by a nucleic acid, either directly or in the form of a precursor. A single amino acid is encoded by a nucleic acid consisting of three nucleotides (so-called codon or base triplet). Each amino acid is encoded by at least one codon. An amino acid may be encoded by different codons, which is known as "codon degeneracy". Amino acids include natural amino acids and non-natural amino acids. Natural amino acids include alanine (three-letter code: ala, one-letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

"Conservative amino acid substitution" refers to the replacement of one amino acid residue with another amino acid residue having a side chain (R group) of similar chemical nature (e.g., charge or hydrophobicity). Generally, conservative amino acid substitutions do not substantially alter the functional properties of the protein. Examples of amino acid classes with a side chain of similar chemical nature include: 1) an aliphatic side chain: glycine, alanine, valine, leucine and isoleucine; 2) an aliphatic hydroxyl side chain: serine and threonine; 3) an amide-containing side chain: asparagine and glutamine; 4) an aromatic side chain: phenylalanine, tyrosine and tryptophan; 5) a basic side chain: lysine, arginine and histidine; and 6) an acidic side chain: aspartic acid and glutamic acid.

A number of amino acids of "conservative amino acid substitutions of VL or VH" is about 1, about 2, about 3, about 4, about 5, about 6, about 8, about 9, about 10, about 11, about 13, about 14, about 15 conservative amino acid substitutions, or a range between any two of these values (inclusive) or any value therein. A number of amino acids of "a heavy chain constant region, a light chain constant region, a heavy chain or a light chain, conservative amino acid substitutions of a first or second polypeptide of a fusion protein" is about 1, about 2, about 3, about 4, about 5, about 6, about 8, about 9, about 10, about 11, about 13, about 14, about 15, about 18, about 19, about 22, about 24, about 25, about 29, about 31, about 35, about 38, about 41, about 45 conservative amino acid substitutions, or a range between any two of these values (inclusive) or any value therein. The term "isolated" as used herein with respect to cells, nucleic acids, polypeptides, antibodies and the like, e.g., "isolated" DNA, RNA, polypeptides, antibodies, refers to molecules that are separated from one or more other components, e.g., DNA or RNA, in the natural environment of the cell. The term "isolated" as used herein also refers to nucleic acids or peptides that are substantially free of cellular materials, viral materials or cell culture media when produced by recombinant DNA techniques, or of chemical precursors or other chemicals when chemically synthesized. In addition, "isolated nucleic acid" is intended to include nucleic acid fragments that do not and will not occur in nature. The term "isolated" is also used herein to refer to cells or polypeptides that are separated from other cellular proteins or tissues. Isolated polypeptides are intended to include both purified and recombinant polypeptides. Isolated polypeptides, antibodies and the like are usually prepared by at least one purification step. In some embodiments, the purity of the isolated nucleic acids, polypeptides and antibodies is at least about 50%, about 60%, about 70%, about 80%, about 90%, about 95%, about 99%, or within a range between any two of these values (inclusive), or any values therein.

The term "recombinant", with regard to a polypeptide or polynucleotide, is intended to refer to a polypeptide or polynucleotide that does not occur in nature, and non-limiting examples can be combined to produce a polynucleotide or polypeptide that does not normally occur.

"Homology" or "identity" or "similarity" refers to sequence similarity between two peptides or between two nucleic acid molecules. Homology can be determined by comparing the positions that can be aligned in the sequences. When a position of the compared sequences is occupied by the same base or amino acid, then the molecules are homologous at that position. The degree of homology between the sequences is a function of the number of matching or homologous positions shared by the sequences.

"At least 80% identity" refers to about 80% identity, about 81% identity, about 82% identity, about 83% identity, about 85% identity, about 86% identity, about 87% identity, about 88% identity, about 90% identity, about 91% identity, about 92% identity, about 94% identity, about 95% identity, about 98% identity, about 99% identity, or a range between any two of these values (inclusive) or any value therein.

"At least 90% identity" refers to about 90% identity, about 91% identity, about 92% identity, about 93% identity, about 94% identity, about 95% identity, about 92% identity, about 96% identity, about 99% identity, or a range between any two of these values (inclusive) or any value therein. A polynucleotide and a polynucleotide sequence (or polypeptide or antibody sequence) having a certain percentage (e.g., 90%, 95%, 98% or 99%) of "identity" or "sequence identity" to another sequence refers to that when the sequences are aligned, the percentage of bases (or amino acids) in the sequence are the same. This alignment identity percentage or sequence identity can be determined using visual inspection or software programs known in the art, such as the software programs described in Ausubel et al. eds., (2007), Current Protocols in Molecular Biology. Preferably, the alignment is performed using default parameters. One alignment program is BLAST using default parameters, such as BLASTN and BLASTP, both using the following default parameters: Genetic code = standard; filter = none; strand = both; cutoff = 60; expect = 10; Matrix = BLOSUM62; Descriptions = 50 sequences; sort by = HIGH SCORE; Databases = non-redundant; GenBank + EMBL + DDBJ + PDB + GenBank CDS translations + SwissProtein + SPupdate + PIR. Biologically equivalent polynucleotides are polynucleotides having the above specified percentage identity and encoding polypeptides having identical or similar biological activity.

A polynucleotide consists of a specific sequence of four nucleotide bases: adenine (A), cytosine (C), guanine (G), thymine (T)/uracil (U, instead of thymine when the polynucleotide is RNA). A "polynucleotide sequence" can be a letter representation of a polynucleotide molecule. The letter representation can be input into a database in a computer with a central processing unit and used for bioinformatics applications, such as for functional genomics and homology searches.

The terms "polynucleotide" and "oligonucleotide" are used interchangeably to refer to a polymeric form of nucleotides of any length, whether deoxyribonucleotides or ribonucleotides or analogs thereof. The polynucleotide may have any three-dimensional structure and may perform any function, known or unknown. The following are non-limiting examples of polynucleotides: genes or gene fragments (such as probes, primers, EST or SAGE tags), exons, introns, messenger RNA (mRNA), transfer RNA, ribosomal RNA, ribozymes, cDNA, dsRNA, siRNA, miRNA, recombinant polynucleotides, branched polynucleotides, plasmids, vectors, isolated DNA of any sequence, isolated RNA of any sequence, nucleic acid probes and primers. Polynucleotides can include modified nucleotides, such as methylated nucleotides and nucleotide analogs. If present, structural modifications to the nucleotide can be made before or after the assembly of the polynucleotide. The sequence of nucleotides can be interrupted by non-nucleotide components. The polynucleotide may be further modified after polymerization, such as by conjugation with a labeling component. This term also refers to double-stranded and single-stranded molecules. Unless otherwise indicated or required, examples of any polynucleotide disclosed herein include a double-stranded form and each of the two complementary single-stranded forms known or predicted to constitute the double-stranded form.

The term "encoding" as applied to a polynucleotide refers to a polynucleotide known to "encode" a polypeptide. When in its native state or manipulated by methods well known to those skilled in the art, the polypeptide and a fragment thereof can be produced by transcript and/or translation.

"Antibody" and "antigen-binding fragment" refers to a polypeptide or polypeptide complex that specifically recognizes and binds an antigen. The antibody may be an intact antibody and any antigen-binding fragment or single chain thereof. The term "antibody" thus includes any protein or peptide comprising at least a portion of an immunoglobulin molecule that has biological activity for binding to an antigen. The antibody and the antigen-binding fragment include, but are not limited to, Complementarity Determining Regions (CDRs) of a heavy or light chain or a ligand binding portion thereof, heavy chain variable regions (VHs), light chain variable regions (VLs), heavy chain constant regions (CHs), light chain constant regions (CLs), framework regions (FRs) or any portion thereof, or at least a portion of a binding protein, as described in the examples. The CDRs include light chain CDRs (VL CDR1-3) and heavy chain CDRs (VH CDR1-3). The variable regions may comprise the structure FR1-CDR1-FR2-CDR2-FR3-CDR3-FR4.

The term "antibody fragment" or "antigen-binding fragment" refers to a part of an antibody, such as F(ab')2, F(ab)2, Fab', Fab, Fv, scFv, etc. Regardless of its structure, the antibody fragment binds to the same antigen recognized by an intact antibody. The term "antibody fragment" includes aptamers, mirror image isomers, and bivalent antibodies. The term "antigen-binding fragment" also includes any synthetic or genetically engineered protein that functions as an antibody by binding to a specific antigen to form a complex.

"Single chain variable fragment" or "scFv" refers to a fusion protein of a heavy chain variable region (VH) and a light chain variable region (VL) of an immunoglobulin. In some aspects, these regions are linked to a short linker peptide having 10 to about 25 amino acids. The linker may be enriched with glycine to improve flexibility, and enriched with serine or threonine to improve solubility, and may link the N terminus of VH and the C terminus of VL, or vice versa. Although the protein has the constant region removed and the linker introduced, it retains the specificity of the original immunoglobulin. ScFv molecules are generally known in the art and are described, for example, in U.S. patent No. 5,892,019.

The term "antibody" includes a wide variety of polypeptides that can be biochemically distinguished. Those skilled in the art will appreciate that the classes of heavy chains include gamma, mu, alpha, delta, or epsilon (γ, µ, α, δ, or ε), and some subclasses (e.g., γ1-γ4). The nature of this chain determines the "type" of the antibody as IgG, IgM, IgA, IgD or IgE. Immunoglobulin subclasses (isotypes), such as IgG1, IgG2, IgG3, IgG4, IgG5, etc., have been well characterized and the functional specificity imparted is also known. All types of immunoglobulins are within the scope of the present invention. In some embodiments, the immunoglobulin molecule is a IgG species. IgG typically contains two identical light chain polypeptides with a molecular weight of about 23,000 Daltons and two identical heavy chain polypeptides with a molecular weight of about 53,000-70,000. These four chains are connected in a "Y" configuration through disulfide bonds, wherein the light chain starts at the opening of "Y" configuration and extends through the variable region to surround the heavy chain.

Antibodies, antigen-binding fragments, or derivatives disclosed herein include, but are not limited to, polyclonal, monoclonal, multispecific, fully human, humanized, primatized, chimeric antibodies, single chain antibodies, epitope-binding fragments such as Fab, Fab' and F(ab')2, Fd, Fv, single chain Fv (scFv), disulfide-linked Fv (sdFv), fragments comprising VK or VH domains, fragments produced from Fab expression libraries, and anti-idiotype (anti-Id) antibodies. The immunoglobulin or antibody molecules disclosed herein can be of any type (e.g., IgG, IgE, IgM, IgD, IgA and IgY) or class (e.g., IgG1, IgG2, IgG3, IgG4, IgA1 and IgA2) or subclass of immunoglobulin.

Light chains can be classified into kappa (κ) or lambda (λ). Each heavy chain may be connected with a κ or λ light chain. In general, when an immunoglobulin is produced by a hybridoma, a B cell or a genetically engineered host cell, the light and heavy chains are bound by covalent bonds, and the "tail" portions of the two heavy chains are bound by covalent disulfide bonds or non-covalent bonds. In the heavy chains, the amino acid sequence extends from the N terminus at the forked end of the Y configuration to the C terminus at the bottom of each chain. The immunoglobulin κ light chain variable region is Vκ; the immunoglobulin λ light chain variable region V_{λ}.

Both the light and heavy chains are divided into structurally and functionally homologous regions. The terms "constant" and "variable" are used in accordance with function. The light chain variable region (VL) and heavy chain variable region (VH) determine the antigen recognition and specificity. The light chain constant region and heavy chain constant regions impart important biological properties such as secretion, transplacental movement, Fc receptor binding, complement binding, and the like. By convention, the numbering of amino acids in the constant regions increases as they become further away from the antigen-binding site of the antibody or amino terminus. The N-terminal portion is the variable region, and the C-terminal portion is the constant region; the CH3 and CL domains actually comprise the carboxyl termini of the heavy chain and light chain, respectively.

As described above, the variable regions enable the antibody to selectively recognize and specifically bind to an epitope on the antigen. Specifically, the VL domain and VH domain of an antibody, or a subset of Complementarity Determining Regions (CDRs), combine to form a variable region defining a three-dimensional antigen-binding site. The antibody quaternary structure forms the antigen-binding site present at the end of each arm of the Y configuration.

More specifically, the antigen-binding sites are defined by three CDRs in each of the VH and VL (i.e., VH CDR1, VH CDR2, VH CDR3, VL CDR1, VL CDR2 and VL CDR3). In certain cases, such as certain camelid-derived immunoglobulin molecules or camelid immunoglobulin-based engineered immunoglobulin molecules, an intact immunoglobulin molecule may consist of only heavy chains, without light chains, see, for example, Hamers-Casterman et al.,Nature,363:446-448 (1993).

In naturally occurring antibodies, the six "Complementarity Determining Regions" or "CDRs" present in each antigen-binding domain are short, non-contiguous, antigen-specific binding amino acid sequences that form the antigen-binding domain, assuming that the antibody is present in its three-dimensional configuration in an aqueous environment. The remaining amino acids in the antigen-binding domain, referred to as the "framework" region, exhibit little intermolecular variability. Most of the framework regions adopt a β-sheet conformation, with the CDRs forming a loop structure connected to, or in some cases forming part of, the β-sheet structure. Thus, the framework regions position the CDRs in a correct orientation by interchain non-covalent interactions through forming a scaffold. The antigen-binding domain with the specifically positioned CDRs forms a surface complementary to an epitope on the antigen that facilitates non-covalent binding of the antibody to its antigenic epitope. For given heavy or light chain variable region, amino acids comprising the CDRs and the framework regions may be identified by one of ordinary skills in the art according to known method (see, e.g., Kabat, E., et al., U.S. Department of Health and Human Services, Sequences of Proteins of Immunological Interest, (1983) and Chothia and Lesk, J. Mol. Biol., 196:901-917 (1987)).

Where the terms used and/or accepted in the art have two or more definitions, the definitions of the terms used herein include all of these meanings unless explicitly indicated as opposite. One specific example is the use of the term "Complementarity Determining Regions" ("CDRs") to describe non-contiguous antigen-binding sites found within the variable regions of heavy and light chain polypeptides. This specific region is described in Kabat et al., U.S. Dept. of Health and Human Services, Sequences of Proteins of Immunological Interest (1983) and Chothiaet al., J. Mol. Biol. 196:901-917 (1987), which is incorporated herein by reference in its entirety.

CDRs defined according to Kabat and Chothia systems include overlaps or subsets of amino acid residues when compared to each other. Nevertheless, it is within the scope of the present invention to apply any definition to refer to the CDRs of an antibody or a variant thereof. The exact number of residues comprising a specific CDR will vary according to the sequence and size of the CDR. Those skilled in the art can generally determine which specific residues a CDR comprises based on the variable region amino acid sequence of an antibody.

Kabat et al. also defines a numbering system applicable to the variable region sequence of any antibody. One of ordinary skill in the art can apply the "Kabat numbering" system to any variable region sequence without depending on other experimental data beyond the sequence itself. "Kabat numbering" refers to the numbering system proposed by Kabat et al., U.S. Dept. of Health and Human Services in "Sequence of Proteins of Immunological Interest" (1983). EU numbering system can be also applicable to the antibody.

The antibodies disclosed herein may be derived from any animal, including birds and mammals. Preferably, the antibody is derived from a human, a mouse, a donkey, a rabbit, a goat, a camel, a llama, a horse, or a chicken source. In another embodiment, the variable region may come from a condricthoid source (e.g., from a shark).

"Heavy chain constant region" includes amino acid sequences derived from immunoglobulin heavy chains. The polypeptide comprising a heavy chain constant region comprises at least one of a CH1 domain, a hinge (e.g., upper, middle, and/or lower hinge region) domain, a CH2 domain, a CH3 domain, or a variant or a fragment. For example, the antibody or the antigen-binding fragment disclosed herein comprise a CH1 domain; comprises a CH1 domain, at least a portion of a hinge region, and a CH2 domain; comprises a CH1 domain and a CH3 domain; comprises a CH1 domain and at least a portion of a hinge region and a CH3 domain; or comprises a CH1 domain, at least a portion of a hinge region and a CH2 domain and a CH3 domain. In another embodiment, the antibody or the antigen-binding fragment disclosed herein comprises a CH3 domain. In addition, the antibody or the antigen-binding fragment used in the present invention may lack a portion of or all of the CH2 domain. As described above, it should be appreciated by those of ordinary skill in the art that the heavy chain constant regions may be modified such that the amino acid sequence of the immunoglobulin molecules in which they naturally occur is changed.

The heavy chain constant region of the antibody may be derived from different immunoglobulin molecules. For example, the heavy chain constant region of a polypeptide may include a CH1 domain derived from a IgG₁ molecule and a hinge region derived from a IgG₃ molecule. In another embodiment, the heavy chain constant region may include a hinge region derived partially from a IgG₁ molecule and partially from a IgG₃ molecule. In another embodiment, a portion of the heavy chain may include a chimeric hinge region derived partially from a IgG₁ molecule and partially from a IgG₄ molecule.

"Light chain constant region" includes the amino acid sequence from the light chain of an antibody. Preferably, the light chain constant region comprises at least one of a constant κ domain or a constant λ domain. A "light chain-heavy chain pair" refers to a collection of light and heavy chains that can form dimers through disulfide bonds between the CL domain of the light chain and the CH1 domain of the heavy chain.

As described above, the structures and three-dimensional configurations of the subunits in the constant regions of various immunoglobulin classes are well known. "VH domain" includes the variable domain at the amino terminus of an immunoglobulin heavy chain, and "CH1 domain" includes the first constant region (mostly at the amino terminus) of an immunoglobulin heavy chain. The CH1 domain is adjacent to the VH domain, and is the amino terminus of the hinge region of the immunoglobulin heavy chain molecule. The CH2 domain is not closely paired with other domains, but rather two N-linked branched carbohydrate chains are inserted between the two CH2 domains of an intact native IgG molecule. The CH3 domain extends from the CH2 domain to the C terminus of the IgG molecule comprises about 108 residues. "Hinge region" includes a portion of the heavy chain region connecting the CH1 domain and CH2 domain. The hinge region comprises about 25 residues and is flexible, thereby enabling independent movement of the two N-terminal antigen-binding regions. The hinge region can be subdivided into three distinct domains: upper, middle and lower hinge domains (Roux et al., J. Immunol., 161:4083 (1998)).

"Disulfide bond" refers to a covalent bond formed between two sulfur atoms. The thiol group of cysteine can form a disulfide bond or a bridge with a second thiol group. In most naturally occurring IgG molecules, the CH1 and CL regions are connected by disulfide bonds, and the two heavy chains are connected by two disulfide bonds at corresponding positions 239 and 242 in the Kabat numbering system (or positions 226 and 229 in the EU numbering system).

A "chimeric antibody" refers to any antibody in which the variable region of the antibody is obtained or derived from a first species, and constant region thereof (which may be intact, partial, or modified) is derived from a second species. In certain embodiments, the variable region is derived from a non-human source (e.g., mouse or primate) and the constant region is derived from a human source.

"Specifically bind to" or "specific for" generally refers to that an antibody or an antigen-binding fragment and a specific antigen bind to an epitope through its antigen-binding domain to form a relatively stable complex. "Specificity" can be expressed by relative affinity of an antibody or an antigen-binding fragment for binding to a specific antigen or epitope. For example, an antibody "A" can be considered to have a higher specificity for an antigen than an antibody "B" if the antibody "A" has a greater relative affinity for the antigen than the antibody "B". Specific binding can be described by the equilibrium dissociation constant (KD). A smaller KD means a tighter binding. Methods for determining whether two molecules specifically bind to each other are well known in the art, and include, for example, equilibrium dialysis, surface plasmon resonance, biofilm layer interferometry, and the like. An antibody that "specifically binds to" a spike protein includes an antibody that has an equilibrium dissociation constant KD for the spike protein of less than or equal to about 100 nM, less than or equal to about 10 nM, less than or equal to about 5 nM, less than or equal to about 1 nM, or less than or equal to about 0.5 nM.

"Treatment" refers to both therapeutic treatment and prophylactic or preventative measures, the purpose of which is to prevent, slow down, ameliorate, or halt undesirable physiological changes or disorders, such as the progression of a disease, including, but not limited to, alleviation of symptoms, diminishment of extent of disease, stabilized (i.e., not worsening) state of disease, delay or slowing of disease progression, amelioration, palliation, alleviation, or elimination (whether partial or total), prolongation of life expectancy in the absence of treatment, and the like, as either detectable or undetectable. Patients in need of treatment include patients already with a condition or disorder, patients susceptible to a condition or disorder, or patients in need of prevention of the condition or disorder, patients who may or are expected to benefit from administration of the antibody or the pharmaceutical composition disclosed herein for detection, diagnostic procedures, and/or treatment.

"Patient" refers to any mammal in need of diagnosis, prognosis or treatment, including humans, dogs, cats, rabbits, rats, mice, horses, cattle and the like.

### Antibody and Fusion Protein

The present invention provides an antibody or an antigen-binding fragment thereof having high affinity for spike proteins. The antibody or the antigen-binding fragment exhibit potent binding activity and are useful for therapeutic and diagnostic purposes. For example, these antibodies or antigen-binding fragments can prevent fusion of SARS-CoV or SARS-CoV-2 viral particles with cell membranes, and mediates phagocytosis and clearance of the virus particles by immune cells.

In some embodiments, a heavy chain of the antibody comprises a sequence having an amino acid sequence set forth in any one of SEQ ID NOs: 53-56 or 108, and/or a light chain of the antibody comprises a sequence having an amino acid sequence set forth in SEQ ID NO: 57.

In some embodiments, a heavy chain of the antibody comprises a sequence having an amino acid sequence set forth in SEQ ID NO: 53, and a light chain of the antibody comprises a sequence having an amino acid sequence set forth in SEQ ID NO: 57. In some embodiments, a heavy chain of the antibody comprises a sequence having an amino acid sequence set forth in SEQ ID NO: 54, and a light chain of the antibody comprises a sequence having an amino acid sequence set forth in SEQ ID NO: 57. In some embodiments, a heavy chain of the antibody comprises a sequence having an amino acid sequence set forth in SEQ ID NO: 55, and a light chain of the antibody comprises a sequence having an amino acid sequence set forth in SEQ ID NO: 57. In some embodiments, a heavy chain of the antibody comprises a sequence having an amino acid sequence set forth in SEQ ID NO: 56, and a light chain of the antibody comprises a sequence having an amino acid sequence set forth in SEQ ID NO: 57. In some embodiments, a heavy chain of the antibody comprises a sequence having an amino acid sequence set forth in SEQ ID NO: 108, and a light chain of the antibody comprises a sequence having an amino acid sequence set forth in SEQ ID NO: 57.

The present invention further provides a fusion protein. The antibody part or the antigen-binding fragment in the fusion protein has high affinity for the spike protein of SARS-CoV and SARS-CoV-2, and these antibodies can specifically bind to the spike protein; 6-HB interference polypeptides in the fusion protein can interfere with the formation of "six-helix bundle (6-HB)". The fusion protein exhibit potent binding activity and are useful in therapeutic and diagnostic purpose. For example, these antibodies or antigen-binding fragments in the fusion protein can prevent the fusion of SARS-CoV and SARS-CoV-2 viral particles with cell membranes, and mediate phagocytosis and clearance of the virus by immune cells.

In some embodiments, a C-terminus of a heavy chain (i.e., the CH3 terminus) of an antibody described herein is covalently linked to the 6-HB interference polypeptide via a linker. In some embodiments, a C-terminus of a light chain (i.e., the CL terminus) of an antibody described herein is covalently linked to the 6-HB interference polypeptide via a linker.

The sequences of the antibody, the antigen-binding fragment, and the fusion proteins disclosed in the embodiments of the present invention are shown in Tables 1-3, wherein the sequences include a CDR region, an FR region, heavy and light chain constant regions, heavy and light chain variable regions, as well as a linker, a 6-HB interference polypeptides and the like of the antibody or the antigen-binding fragment; the CDR region and FR region for the antibody or the antigen-binding fragment may also be divided according to the Kabat definition as shown in Table 2.

In some embodiments, the fusion protein disclosed herein comprises a 6-HB interference polypeptide and an antibody or an antigen-binding fragment, a C-terminus of a heavy chain (or a heavy chain fragment) or a C-terminus of a light chain of the antibody is covalently linked, via a linker (set forth in SEQ ID NO: 59), to the 6-HB interference polypeptide, wherein the 6-HB interference polypeptide comprises a sequence set forth in SEQ ID NO: 58, a sequence having at least 90% identity to the sequence set forth in SEQ ID NO: 58, or an amino acid sequence having one or more conservative amino acid substitutions as compared to the sequence set forth in SEQ ID NO: 58.

In some embodiments, a C-terminus of a heavy chain (i.e., the CH3 terminus) of the antibody is covalently linked, via a linker as set forth in SEQ ID NO: 59, to a 6-HB interference polypeptide, wherein the 6-HB interference polypeptide comprises the sequence set forth in SEQ ID NO: 58. In some embodiments, a C-terminus of a light chain (i.e., the CL terminus) of the antibody is covalently linked, via a linker set forth in SEQ ID NO: 59, to a 6-HB interference polypeptide, wherein the 6-HB interference polypeptide comprises the sequence set forth in SEQ ID NO: 58.

In some embodiments, the fusion protein disclosed herein comprises a 6-HB interference polypeptide, as well as an antibody or an antigen-binding fragment, wherein the antibody or the antigen-binding fragment comprises a VH set forth in SEQ ID NO: 46, 47, 48, 49 or 107, a VL set forth in SEQ ID NO: 50, or respective biological equivalents thereof. A biological equivalent of the VH or the VL is a sequence comprising specific amino acids, i.e., the sequence identity of the sequence as a whole is about 80%, about 85%, about 90%, about 95%, about 98%, about 99%, or within a range between any two of these values (inclusive), or any values therein.

In some embodiments, the fusion protein comprises an antibody and a 6-HB interference polypeptide linked via a linker, a heavy chain of the antibody comprises a sequence having an amino acid sequence of amino acids at positions 1-450 of SEQ ID NO: 53, and a light chain of the antibody comprises a sequence having an amino acid sequence set forth in SEQ ID NO: 57; a C-terminus of the heavy chain (i.e., CH3 terminus) or a C-terminus of the light chain (i.e., CL terminus) of the antibody is covalently linked, via a linker set forth in SEQ ID NO: 59, to the 6-HB interference polypeptide, wherein the 6-HB interference polypeptide comprises a sequence set forth in SEQ ID NO: 58.

In some embodiments, the fusion protein comprises an antibody and a 6-HB interference polypeptide linked via a linker, a heavy chain of the antibody comprises a sequence having an amino acid sequence of amino acids at positions 1-450 of SEQ ID NO: 54, and a light chain of the antibody comprises a sequence having an amino acid sequence set forth in SEQ ID NO: 57; a C-terminus of the heavy chain (i.e., CH3 terminus) or a C-terminus of the light chain (i.e., CL terminus) of the antibody is covalently linked, via a linker set forth in SEQ ID NO: 59, to the 6-HB interference polypeptide, wherein the 6-HB interference polypeptide comprises a sequence set forth in SEQ ID NO: 58.

In some embodiments, the fusion protein comprises an antibody and a 6-HB interference polypeptide linked via a linker, a heavy chain of the antibody comprises a sequence having an amino acid sequence of amino acids at positions 1-450 of SEQ ID NO: 55, and a light chain of the antibody comprises a sequence having an amino acid sequence set forth in SEQ ID NO: 57; a C-terminus of the heavy chain (i.e., CH3 terminus) or a C-terminus of the light chain (i.e., CL terminus) of the antibody is covalently linked, via a linker set forth in SEQ ID NO: 59, to the 6-HB interference polypeptide, wherein the 6-HB interference polypeptide comprises a sequence set forth in SEQ ID NO: 58.

In some embodiments, the fusion protein comprises an antibody and a 6-HB interference polypeptide linked via a linker, a heavy chain of the antibody comprises a sequence having an amino acid sequence of amino acids at positions 1-450 of SEQ ID NO: 56, and a light chain of the antibody comprises a sequence having an amino acid sequence set forth in SEQ ID NO: 57; a C-terminus of the heavy chain (i.e., CH3 terminus) or a C-terminus of the light chain (i.e., CL terminus) of the antibody is covalently linked, via a linker set forth in SEQ ID NO: 59, to the 6-HB interference polypeptide, wherein the 6-HB interference polypeptide comprises a sequence set forth in SEQ ID NO: 58.

In some embodiments, the fusion protein comprises an antibody and a 6-HB interference polypeptide linked via a linker, a heavy chain of the antibody comprises a sequence having an amino acid sequence of amino acids at positions 1-450 of SEQ ID NO: 108, and a light chain of the antibody comprises a sequence having an amino acid sequence set forth in SEQ ID NO: 57; a C-terminus of the heavy chain (i.e., CH3 terminus) or a C-terminus of the light chain (i.e., CL terminus) of the antibody is covalently linked, via a linker set forth in SEQ ID NO: 59, to the 6-HB interference polypeptide, wherein the 6-HB interference polypeptide comprises a sequence set forth in SEQ ID NO: 58.

In some embodiments, the antibody, or the fusion protein such as an antibody therein may further be linked to an amino acid sequence or one or more modifying groups. For example, the antibody, or the fusion protein such as an antibody therein disclosed herein may comprise a flexible linker sequence, or may be modified to add a functional group (e.g., PEG, a drug, a toxin, or a tag).

The antibody, or the fusion protein such as an antibody and an antigen-binding fragment therein disclosed herein further comprises modified derivatives, i.e., derivatives modified by covalent linking of any type of molecule with the antibody, wherein the covalent linking does not prevent the antibody from binding to the epitope. The following examples are included but not by way of limitation, wherein an antibody may be modified by glycosylation, acetylation, pegylation, phosphorylation, amidation, derivatization by known protecting/blocking groups, proteolytic cleavage, attachment to cellular ligands or other proteins, or the like. Any of a number of chemical modifications may be made by techniques in the prior art, including but not limited to specific chemical cleavage, acetylation, formylation, metabolic synthesis of tunicamycin, and the like.

In some embodiments, the antibody, or the fusion protein such as an antibody therein may be conjugated to a therapeutic agent, a prodrug, a peptide, a protein, an enzyme, a virus, a lipid, a biological response modifier, an agent, or PEG.

The antibody, or the fusion protein such as an antibody therein may be conjugated or fused with a therapeutic agent, which may comprise a detectable label, such as a radiolabel, an immunomodulator, a hormone, an enzyme, an oligonucleotide, a photoactive therapeutic agent, a diagnostic agent, a cytotoxic agent, an ultrasound enhancing agent, a nonradioactive label, and compositions thereof, and other such agents known in the art.

The antibody, or the fusion protein such as an antibody therein may be detectably labeled by coupling to a chemiluminescent compound. The presence of the chemiluminescent-labeled antibody is then determined by detecting the luminescence that occurs during the course of chemical reactions. Examples of chemiluminescent-labeled compounds include luminol, isoluminol, aromatic acridinium esters, imidazole, acridinium salts and oxalate esters.

### Polynucleotide Encoding Antibody and Fusion Protein and Method for Preparing Antibody

The present invention further discloses a polynucleotide or a nucleic acid molecule encoding the antibody, the antigen-binding fragment, the fusion protein and a derivative thereof of the present invention. The polynucleotide disclosed herein may encode a heavy chain, a light chain, a heavy chain variable region, a light chain variable region, an Fc region, a portion of a heavy chain variable region or a portion of a light chain variable region, or a fusion protein. The methods for preparing the antibody and the fusion protein is well known in the art and is described herein. In certain embodiments, the variable and constant regions of the antibody and the antigen-binding fragment disclosed herein are of fully human. The fully human antibody and antigen-binding fragment can be prepared using techniques disclosed in the art and described herein. For example, the fully human antibody against a specific antigen can be prepared by administering the antigen to transgenic animals that have been modified to produce the fully human antibody in response to antigen challenge. Exemplary techniques that can be used to prepare such an antibody are found in U.S. Patent Nos. 6,458,592 and 6,420,140; which are incorporated herein by reference in their entireties.

In certain embodiments, the prepared antibody and fusion protein do not elicit a deleterious immune response in the animal, e.g., human, to be treated. In one embodiment, the antibody, the antigen-binding fragment, the fusion protein or the derivative disclosed herein are modified using techniques well known in the art to reduce the immunogenicity. For example, the antibody can be humanized, primatized or deimmunized, or a chimeric antibody can be prepared. Such antibodies are derived from non-human antibodies, typically murine or primate antibodies, which retain or substantially retain the antigen-binding properties of the parent antibody but are less immunogenic in humans. This can be accomplished by a variety of methods, including: (a) grafting an entire variable region of a non-human origin to a constant region of a human origin to produce a chimeric antibody; (b) grafting at least a portion of one or more non-human Complementarity Determining Regions (CDRs) to framework regions and a constant region of a human origin, with or without retention of critical framework residues; or (c) grafting an entire variable region of a non-human origin, but "hiding" the surface residues by replacing them with portions of a human-like origin. Generally, framework residues in the human framework regions will be substituted by corresponding residues from the CDR donor antibody, preferably residues that may improve the antigen binding. Such framework substitutions can be identified by methods well known in the art, for example, by modeling the interaction of the CDR and framework residues to identify framework residues that play an important role in antigen binding and by sequence alignment to identify aberrant framework residues at particular positions. (see, U.S. Pat. No. 5,585,089 and Riechmann et al., Nature 332:323 (1988); which are incorporated herein by reference in their entireties). The antibody can be humanized by a variety of techniques well known in the art, such as CDR grafting (Pat. Nos. EP 239,400 and WO 91/09967, and U.S. Pat. Nos. 5,225,539, 5,530,101 and 5,585,089), repair or surface rearrangement (Pat. Nos. EP592,106 and EP519,596, and Padlan, et al., Molecular Immunology 28(4/5):489-498 (1991); Studnicka et al., Protein Engineering 7(6):805-814 (1994); Roguska, et al., Proc. Natl. Sci. USA 91:969-973 (1994)), and chain rearrangement (U.S. Pat. No. 5,565,332), which are incorporated herein by reference in their entireties.

Deimmunization may also be used to reduce the immunogenicity of the antibody. In the present invention, the term "deimmunization" includes the alteration of the antibody to modify T cell epitopes (see, e.g., Pat. Nos. WO/9852976 A1 and WO/0034317 A2). For example, a heavy chain variable region sequence and a light chain variable region sequence from the original antibody are analyzed, and a "map" of human T cell epitopes from each variable region is generated, showing the positions of the epitopes relative to the Complementarity Determining Regions (CDRs) and other critical residues within the sequence. Individual T cell epitopes from the T cell epitope map are analyzed to identify alternative amino acid substitutions with a lower risk of altering antibody activity. A series of alternative heavy chain variable region sequences and light chain variable region sequences comprising combinations of amino acid substitutions are designed and subsequently incorporated into a series of binding polypeptides. The genes of intact heavy and light chains comprising the modified variable regions and human constant regions are cloned into an expression vector, and the plasmid is then transferred into a cell line to produce an intact antibody. The antibodies are compared in appropriate biochemical and biological experiments to identify the optimal antibody.

The binding specificity of the antibody, or an antibody and an antigen-binding fragment in the fusion protein disclosed herein can be detected by an *in vitro* assay, such as co-immunoprecipitation, radioimmunoassay (RIA) or enzyme-linked immunosorbent assay (ELISA).

Or, the technique for producing a single chain unit (U.S. Pat. No. 4,694,778; Bird, Science 242:423-442 (1988), Huston et al., Proc. Natl. Acad. Sci. USA 55:5879-5883 (1988) and Ward et al., Nature 334:544-554 (1989)) can be used to produce the single chain unit disclosed herein. Single chain fusion peptides are produced by amino acid bridging the heavy and light chain fragments of the Fv region to form a single chain unit. The technique of assembling a functional Fv fragment in *E. coli* (Skerra et al., Science 242:1038-1041 (1988)) can also be used.

Examples of techniques that can be used to produce a single chain Fv (scFv) and an antibody include, for example, those described in U.S. Patent Nos. 4,946,778 and 5,258,498, and Huston et al., Methods in Enzymology, 203:46-88 (1991), Shu et al., Proc. Natl. Sci. USA, 90:1995-1999 (1993) and Skerra et al., Science, 240:1038-1040 (1988). For certain use, including *in vivo* use of antibodies in humans and *in vitro* detection assays, a chimeric antibody, a humanized antibody or a fully human antibody may be used. Chimeric antibodies are molecules in which different portions of the antibody are derived from different animal species, such as antibodies having variable regions of a murine monoclonal antibody and constant regions of a human immunoglobulin. Methods for producing chimeric antibodies are known in the art, see Morrison, Science 229:1202 (1985); Oi et al., BioTechniques 4:214 (1986); Gillies et al., J. Immunol. Methods 125:191-202 (1989); Neuberger et al., Nature 372:604-608 (1984); Takeda et al., Nature 314:452-454 (1985); and U.S. Patent Nos. 5,807,715, 4,816,567 and 4,816,397, which are incorporated herein by reference in their entireties.

In addition, another efficient method for producing recombinant antibodies, which in particular is capable of producing primate antibodies comprising monkey variable region and human constant region sequences, is disclosed in Newman, Biotechnology 10:1455-1460 (1992), which is incorporated herein by reference in its entirety. Furthermore, this technique is also described in commonly assigned U.S. Pat. Nos. 5,658,570, 5,693,780 and 5,756,096, each of which is incorporated herein by reference in its entirety.

The antibodies can be prepared by a variety of methods known in the art, including phage display methods using antibody libraries from immunoglobulin sequences. Reference may also be made to U.S. Pat. Nos. 4,444,887 and 4,716,111, and PCT Publication Nos. WO98/46645, WO98/50433, WO98/24893, WO98/16654, WO96/34096, WO96/33735 and WO91/10741, each of which is incorporated herein by reference in its entirety.

Fully human antibodies are particularly desirable for treating human patients. Fully human antibodies can be prepared by a variety of methods known in the art, for example, human antibodies can also be produced by transgenic mice that cannot express functional endogenous immunoglobulins but express human immunoglobulin genes. For example, human heavy and light chain immunoglobulin gene complexes can be introduced randomly or by homologous recombination into mouse embryonic stem cells. Alternatively, in addition to the human heavy and light chain genes, human variable, constant and diversity regions can be introduced into mouse embryonic stem cells. Mouse heavy and light chain immunoglobulin genes can be disabled by homologous recombination separately or simultaneously through introducing human immunoglobulin loci. For example, homozygous deletion of the JH region can prevent the production of endogenous antibodies. The modified embryonic stem cells are expanded and microinjected into blastocysts to produce chimeric mice. The chimeric mice are then bred to produce homozygous progeny that express human antibodies. The transgenic mice are immunized by conventional methods with selected antigens, e.g., all or part of the desired polypeptide targets. Antigen-targeting monoclonal antibodies can be obtained from the immunized transgenic mice using conventional hybridoma techniques. The human immunoglobulin transgenes carried by the transgenic mice rearrange during B cell differentiation, resulting in class switching and somatic mutation. Accordingly, using this technique, IgG, IgA, IgM, and IgE antibodies that are useful for therapy can be produced. For a review related to this technique for producing fully human antibodies, see Lonberg and Huszar, Int. Rev. Immunol. 73:65-93 (1995). For a detailed discussion of this technique for producing fully human antibodies and human monoclonal antibodies and procedures for producing such antibodies, see PCT Publication Nos. WO98/24893, WO96/34096 and WO96/33735, and U.S. Pat. Nos. 5,413,923, 5,625,126, 5,633,425, 5,569,825, 5,661,016, 5,545,806, 5,814,318 and 5,939,598, which are incorporated herein by reference in their entireties.

Fully human antibodies recognizing selective epitopes can also be produced using a technique known as "guided selection". In this method, a selected non-human monoclonal antibody such as a mouse antibody is used to guide the screening of fully human antibodies that recognize the same epitope (see U.S. Pat. No. 5,565,332, the entire contents of which are incorporated herein by reference).

In another embodiment, DNA encoding the desired monoclonal antibody can be isolated and sequenced using conventional methods (e.g., using oligonucleotide probes that can specifically bind to genes encoding the heavy and light chains of a murine antibody). Isolated and subcloned hybridoma cells can be preferred sources of such DNA. Once isolated, the DNA can be inserted into an expression vector and then transfected into prokaryotic or eukaryotic host cells such as *E. coli* cells, simian COS cells, Chinese hamster ovary (CHO) cells, or myeloma cells that do not produce immunoglobulins. The isolated DNA (which may be synthetic as described herein) can be used to prepare sequences of the constant and variable regions of an antibody, as described in U.S. Pat. No. 5,658,570, which is incorporated herein by reference in its entireties. This method extracts RNA from the selected cells and conversion to cDNA, followed by amplification by PCR using Ig-specific primers. Suitable probes for this purpose are also described in U.S. Pat. No. 5,658,570.

In addition, using conventional recombinant DNA techniques, one or more CDRs of the antibody disclosed herein can be inserted into framework regions, e.g., into human framework regions to construct a humanized non-fully human antibody. The framework regions may be naturally occurring or consensus framework regions, preferably human framework regions (see Chothia et al., J. Mol. Biol. 278:457-479 (1998), in which a range of human framework regions are listed). Some polynucleotides may encode antibodies produced by the framework region and CDR combinations that specifically bind to at least one epitope of an antigen of interest. One or more amino acid substitutions may be made within the framework regions, and amino acid substitutions capable of improving the binding of the antibody to the antigen thereof may be selected. Additionally, substitution or deletion of one or more cysteine residues in the variable regions involved in interchain disulfide bond formation can be made in this manner, thereby producing an antibody molecule lacking one or more interchain disulfide bonds. Other alterations to the polynucleotides made within the technology scope of the art are also encompassed by the present invention.

Antibody-producing cell lines can be selected, constructed and cultured using techniques well known to those skilled in the art. These techniques are described in various laboratory manuals and main publications. In this aspect, for the techniques suitable for use in the present invention described below, reference is made to Current Protocols in Immunology, Coligan et al., Eds., Green Publishing Associates and Wiley-Interscience, John Wiley and Sons, New York (1991), which is incorporated herein by reference in its entirety, including the supplements.

In some embodiments, DNA encoding the antibody, the antigen-binding fragment or the fusion protein can be designed and synthesized according to the amino acid sequence of the antibody by conventional methods, the antigen-binding fragment or the fusion protein described herein, inserted into an expression vector, and transfected into a host cell. The transfected host cell is then cultured in a medium to produce the antibody, the antigen-binding fragment or the fusion protein. In some embodiments, the expression vector comprises at least one promoter element, an antibody, an antigen-binding fragment or a fusion protein coding sequence, a transcription termination signal, and a polyA tail. Other elements include enhancers, Kozak sequences, and donor and recipient sites flanking the inserted sequence for RNA splicing. Efficient transcription can be obtained by early and late promoters of SV40, long terminal repeats from retroviruses such as RSV, HTLV1 and HIVI, and early promoters of cytomegalovirus, and promoters from other cells such as actin promoter can also be used. Suitable expression vectors may include pIRES 1neo, pRetro-Off, pRetro-On, PLXSN, or Plncx, pcDNA3.1 (+/-), pcDNA/Zeo (+/-), pcDNA3.1/Hygro(+/-), PSVL, PMSG, pRSVcat, pSV2dhfr, pBC12MI, pCS2, etc. Commonly used mammalian cells include 293 cells, Cos1 cells, Cos7 cells, CV1 cells, murine L cells, CHO cells and the like.

In some embodiments, the inserted gene fragment should comprise a screening label, common ones of which include screening genes such as dihydrofolate reductase, glutamine synthetase, neomycin resistance and hygromycin resistance, to facilitate the screening and separation of cells that have been successfully transfected. The constructed plasmid is transfected to a host cell without the above genes, and the successfully transfected cells are cultured in a large quantity in a selective culture medium to produce the desired target protein.

In addition, mutations can be introduced in the nucleotide sequence encoding the antibody, the antigen-binding fragment or the fusion protein disclosed herein using standard techniques known to those skilled in the art, including but not limited to site-directed mutations resulting in amino acid substitutions and PCR-mediated mutations. The variant (including derivative) encodes a substitution of less than 50 amino acids, a substitution of less than 40 amino acids, a substitution of less than 30 amino acids, a substitution of less than 25 amino acids, a substitution of less than 20 amino acids, a substitution of less than 15 amino acids, a substitution of less than 10 amino acids, a substitution of less than 5 amino acids, a substitution of less than 4 amino acids, a substitution of less than 3 amino acids or a substitution of less than 2 amino acids relative to the VH CDR1, VH CDR2 and VH CDR3 of the original heavy chain variable region and the VL CDR1, VL CDR2 or VL CDR3 of the light chain variable region. Alternatively, mutations can be introduced randomly along all or part of the coding sequence, for example by saturation mutagenesis, and the resulting mutants can be screened for the biological activity to identify mutants that retain the activity.

In some embodiments, the substitutions described herein are conservative amino acid substitutions.

### Therapeutic Method

The present invention further provides a treatment method and use. In some embodiments, provided is a method for treating or ameliorating SARS or COVID-19, wherein the method comprises administering to a patient an effective dose of the antibody, the antigen-binding fragment or the fusion protein. In some embodiments, provided is use of the antibody or the fusion protein in the treatment or amelioration of SARS or COVID-19. In some embodiments, provided is use of the antibody or the fusion protein in the manufacture of a medicament for treating or ameliorating SARS or COVID-19. In some embodiments, the patient is a patient suspected of being infected with SARS-CoV or SARS-CoV-2 virus. In some embodiments, the patient is a patient who has been in contact with SARS-CoV or a SARS-CoV-2 virus carrier. In some embodiments, the patient is diagnosed with infection with SARS-CoV or SARS-CoV-2 virus. In some embodiments, the patient is a patient with mild symptoms. In some embodiments, the patient is a patient with severe symptoms. In some embodiments, the patient has symptoms of fever, cough, hypotension, hypoxia, and/or acute respiratory distress syndrome (ARDS).

The specific dosage and regimen for any particular patient will depend upon a variety of factors including the particular antibody, the antigen-binding fragment or the fusion protein, or derivative used, the age and body weight of the patient, general health condition, sex and diet, and the time of administration, frequency of excretion, drug combination and the severity of the particular disease being treated. These factors are judged by medical caregivers included within the scope of those of ordinary skills in the art. The dosage will also depend on the individual patient to be treated, the route of administration, the type of the formulation, the nature of the compound used, the severity of the disease and the efficacy desired. The dosage employed can be determined by pharmacological and pharmacokinetic principles well known in the art.

The modes of administration for the antibody, the antigen-binding fragment, the fusion protein, or the derivative include, but are not limited to, intradermal, intramuscular, intraperitoneal, intravenous, subcutaneous, nasal, epidural and oral injection. The pharmaceutical composition may be administered by any convenient route, e.g., by infusion or bolus injection, by absorption through epithelial or cutaneous mucosa (e.g., oral mucosa, rectal and intestinal mucosa), and may be co-administered with other biologically active agents. Thus, the pharmaceutical composition comprising the antibody, the antigen-binding fragment or the fusion protein disclosed herein can be administered orally, rectally, parenterally, intracisternally, intravaginally, intraperitoneally, topically (e.g. by powder, ointment, drop or transdermal patch) or buccally, or by oral or nasal spray.

The term "parenteral" as used herein refers to modes of administration including intravenous, intramuscular, intraperitoneal, intrasternal, subcutaneous and intraarticular injections and infusions. The mode of administration may be systemic or local.

In some embodiments, the composition disclosed herein comprises a nucleic acid or a polynucleotide encoding a protein, which can be administered *in vivo* to facilitate expression of the encoded protein by constructing the nucleic acid or the polynucleotide as part of a suitable nucleic acid expression vector; and a part of the vector is administered to be intracellular by the following methods, for example, by using a retroviral vector (see U.S. Pat. No. 4,980,286), by direct injection, by using microprojectile bombardment (e.g., gene gun; Biolistic, Dupont), by coating with lipids or cell surface receptors or transfection reagents, or by ligation with homeobox-like peptides with known capability of entering the nucleus (see, e.g., Joliot et al., 1991, Proc. Natl. Acad. Sci. USA 88:1864-1868). Alternatively, the nucleic acid can be introduced into the cell and integrated into the host cell DNA for expression by homologous recombination.

In some embodiments, the antibody, the antigen-binding fragment or the fusion protein of the present invention is administered to a patient at a dose of 0.01 mg/kg to 100 mg/kg of patient body weight, or 0.1 mg/kg to 20 mg/kg of patient body weight. A second or more doses of the antibody or the antigen-binding fragment or the fusion protein may be administered subsequently to the initial dose, at about the same or less dose as the initial dose, wherein the administration at the subsequent dose may be separated by at least 1 to 3 days; or at least one week. The dose and frequency for administration of the antibody or the fusion protein disclosed herein can be reduced or by enhancing the uptake and tissue penetration ability (e.g., into the brain) of the antibody or the fusion protein through modifications such as lipidation.

Various known delivery systems can be used for the administration of the antibody, the antigen-binding fragment or the fusion proteins or the derivative, or the polynucleotide encoding the same disclosed herein, e.g., encapsulation in liposomes, microparticles, microcapsules, recombinant cells capable of expressing the compound, receptor-mediated endocytosis (see, e.g., Wu and Wu, 1987, J. Biol. Chem. 262:4429-4432), construction of nucleic acids as part of a retrovirus or other vectors.

### Combination Therapy

In some embodiments, the antibody, the antigen-binding fragment or the fusion protein of the present invention can be administered in combination with other therapeutic or prophylactic regimens, which includes one or more antibodies, antigen-binding fragments or fusion proteins of the present invention are administered together or in combination with one or more other therapeutic agents or methods. For combination therapy, the antibody, the antigen-binding fragment or the fusion protein may be administered simultaneously or separately with other therapeutic agents. When administered separately, the antibody, the antigen-binding fragment or the fusion protein of the present invention can be administered before or after administration of another additional therapeutic agent.

In some embodiments, the therapeutic agent administered in combination with the antibody, the antigen-binding fragment or the fusion protein of the present invention is at least one of: HIV drugs, antimalarial drugs, RNA polymerase inhibitors, antiviral drugs and monoclonal antibodies. In some embodiments, the HIV drug comprises Lopinavir/Ritonavir, ASC 09/Ritonavir, and Darunavir. In some embodiments, the antimalarial drug comprises chloroquine, hydroxychloroquine, and chloroquine phosphate. In some embodiments, the RNA polymerase inhibitor comprises Remdesivir. In some embodiments, the antiviral drug comprises Arbidol and Favipiravir. In some embodiments, the monoclonal antibody comprises BDB-001. In some embodiments, the antiviral drug is an antibody or an antigen-binding fragment in the fusion protein of the present invention.

Cytokine storm phenomenon occurs in some patients with severe or critical coronavirus pneumonia, and the antibody, the antigen-binding fragment or the fusion protein of the present invention may be used in combination with adalimumab (e.g., 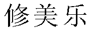 ^{®} (Humira^{®}) and biological analogs thereof such as Abrilada^{™}(adalimumab-afzb), Amjevita (adalimumab-att), Cyltezo^{™} (adalimumab-adbm), Hyrimoz^{™} (adalimumab-adaz), Hulio^{™} , 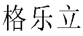 ^{®} (QLETLI^{®}, BAT1406)) or tochilizumab (e.g., 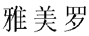 ^{®} (Actemra^{®}/ RoActemra^{®}) and biological analogs thereof such as BAT1806), which may slow inflammatory responses caused by up-regulation of TNF-α expression. In some embodiments, patients treated by the method are diagnosed as infected with the novel coronavirus and having an increase in one or more cytokines including tumor necrosis factor α (TNF-α), IFN-γ, IL-1β, IL-2, IL-4, IL-7, IL-8, IL-10, IL-12p70, IL-13, granulocyte colony stimulating factor (GSCF), interferon inducible protein 10 (IP-10), monocyte chemotactic protein-1 (MCP 1), macrophage inflammatory protein 1α (MIPIA). In some embodiments, the patient treated by the method has increased TNF-α. In some embodiments, one or more cytokines are at least 50% above normal levels. In some embodiments, one or more cytokines are at least 2 times, 3 times or 4 times normal levels. In some embodiments, the patient has symptoms of fever, hypotension, hypoxia, and/or acute respiratory distress syndrome (ARDS) prior to treatment in the present method. In some embodiments, the patient has the lung filled with inflammatory fluid (so-called "white lung") prior to treatment in the present method. In some embodiments, the patient has cytokine release syndrome (CRS) due to cytokine storm prior to treatment in the present method.

In some embodiments, the antibody, the antigen-binding fragment or the fusion protein of the present invention are used in conjunction with ICU therapy. In some embodiments, the antibody, the antigen-binding fragment or the fusion protein of the present invention are administered in combination with *in vitro* ECMO and/or IMV therapy. In some embodiments, the antibody, the antigen-binding fragment or the fusion protein of the present invention are administered in combination with oxygen therapy. In some embodiments, the antibody or the antigen-binding fragments or fusion protein of the present invention are administered in combination with NIV/HFNC therapy. In some embodiments, the patient after treatment has at least a 20%, 30%, 40%, 50%, 60%, 70%, 80%, 90%, or 95% reduction in one or more cytokines over before treatment. In some embodiments, the present method results in recovery of the patient.

### Diagnostic Method

The expression of a spike protein is observed in certain samples, and patients with cells expressing the spike protein are likely to respond to treatment with the antibody, the antigen-binding fragment or the fusion proteins of the present invention. Thus, the antibody, the antigen-binding fragment or the fusion protein of the present invention can also be used for diagnosis and prognosis.

The sample comprising the cells may be obtained from a patient. After the sample is optionally pretreated, the sample can be incubated with the fusion protein (or antibody) of the present invention under a condition in which the antibody (or the fusion protein) can be allowed to interact with the spike protein that may be present in the sample. The presence of a spike protein in a sample can be detected by a method such as ELISA using an antibody, or an antibody in a fusion protein.

The presence of the spike protein (e.g., at amount or concentration) in a sample can be used to diagnose the associated disease, which is an indication that the patient is eligible for treatment with the antibody or the fusion protein, or as an indication that the patient has (or has not) responded to treatment of the disorder. For prognostic methods, one, two or more tests may be performed at a particular stage at the beginning of treatment of a disease to indicate the progress of the treatment.

### Pharmaceutical Composition

The present invention further provides a pharmaceutical composition. Such composition comprises an effective dose of an antibody or a fusion protein and a pharmaceutically acceptable carrier.

In some embodiments, the term "pharmaceutically acceptable" refers to a substance approved by a government regulatory agency or listed in commonly-recognized pharmacopeias for use in animals, and particularly in humans. In addition, the "pharmaceutically acceptable carrier" generally refers to any type of non-toxic solid, semi-solid or liquid filler, diluent, an encapsulating material, a formulation additive, or the like.

The term "carrier" refers to a diluent, adjuvant, excipient or carrier that can be administered to a patient together with the active ingredient. Such pharmaceutical carriers can be sterile liquids, such as water and oils, including petroleum, oils originated from animal, plant or synthesis, such as peanut oil, soybean oil, mineral oil, sesame oil, and the like. Water is a preferred carrier when the pharmaceutical composition is administered intravenously. A saline aqueous solution, a glucose aqueous solution, and a glycerol solution can also be used as a liquid carrier, especially for injection. Suitable pharmaceutical excipients include starch, glucose, lactose, sucrose, gelatin, malt, rice, flour, chalk, silica gel, sodium stearate, glycerol monostearate, talc, sodium chloride, skimmed milk powder, glycerol, propylene, glycol, water, ethanol and the like. If desired, the pharmaceutical composition may also comprise a small amount of a wetting agent, an emulsifier, or a pH buffering agent such as acetates, citrates or phosphates. Antibacterials such as benzyl alcohol or methylparaben, antioxidants such as ascorbic acid or sodium bisulfite, chelating agents such as ethylenediamine tetraacetic acid, and tonicity adjusting agents such as sodium chloride or dextrose are also contemplated. Such pharmaceutical compositions may be in the form of solutions, suspensions, emulsions, tablets, pills, capsules, pulvises, sustained-release formulations and the like. The pharmaceutical composition may be formulated as a suppository using conventional binders and carriers such as triglycerides. Oral formulations may comprise standard carriers such as mannitol, lactose, starch, magnesium stearate, sodium saccharin, cellulose and magnesium carbonate of pharmaceutical grade. Examples of suitable pharmaceutical carriers are described in Remington's Pharmaceutical Sciences by E. W. Martin, which is hereby incorporated herein by reference. Such compositions will contain a clinically effective dose of an antibody or antigen-binding fragment, preferably in purified form, together with an appropriate amount of a carrier, to provide a form suitable for administration to a patient. The formulation should be suitable for the administration mode. The formulation can be encapsulated in an ampoule bottle, a disposable syringe, or a multi-dose vial made of glass or plastic.

In some embodiments, the composition is formulated into a pharmaceutical composition suitable for intravenous injection into a human body according to conventional steps. A composition for intravenous administration is usually a solution in sterile isotonic aqueous buffer. The pharmaceutical composition may also include a solubilizer and a local anesthetic, such as lidocaine, to alleviate the pain at the injection site. In general, the active ingredients are provided in a unit dosage form individually or as a mixture, for example, the active ingredients are encapsulated in sealed containers (such as ampoule bottles or sachets) that can indicate the amount of the active agent, in the form of lyophilized powder or anhydrous concentrate. Where the composition is administered by infusion, the composition can be dispensed in infusion bottles containing sterile, pharmaceutical grade water or saline. Where the composition is administrated by injection, an ampoule bottle containing sterile water or saline for injection can be used, so that the active ingredients can be mixed before administration.

The compound disclosed herein may be formulated in a neutral or salt form. Pharmaceutically acceptable salts include salts of anions derived from acids such as hydrochloric acid, phosphoric acid, acetic acid, oxalic acid, tartaric acid, and the like; and salts of cations derived from sodium, potassium, ammonium, calcium, iron hydroxide, isopropylamine, triethylamine, 2-ethylaminoethanol, histidine, procainecations, and the like.

"About" refers to a general error range for corresponding values as readily understood by those skilled in the relevant art. In some embodiments, "about" mentioned herein refers to the values described and ranges thereof of ± 10%, ± 5% or ± 1%.

"ECMO", i.e., extracorporeal membrane oxygenation, is a medical emergency technology device mainly used to provide continuous extracorporeal respiration and circulation for patients with severe cardiopulmonary failure to sustain the life of the patients.

"ICU", i.e., intensive care unit, can synchronously carry out treatment, nursing and rehabilitation, provides isolation places and equipment for patients with severe illness or coma, and provide services such as optimal nursing, comprehensive treatment, medical and nursing combination, early postoperative rehabilitation, joint nursing exercise treatment and the like.

"IMV", i.e., intermittent mandatory ventilation, implements periodic volume or pressure ventilation based on a preset time interval, i.e., time trigger, during which the patient is allowed to breathe spontaneously at any set basal pressure level during the mandatory ventilation. During spontaneous breathing, the patient may breathe spontaneously with continuous airflow support, or a valve of the machine will be opened on demand to allow the patient to breathe spontaneously. Most ventilators can provide pressure support during spontaneous breathing.

"HFNC", i.e., high-flow nasal cannula oxygen therapy, is an oxygen therapy modality that directly delivers air-oxygen mixed high-flow gas with a certain oxygen concentration to a patient through a nasal obstruction catheter that does not need to be sealed. As a form of noninvasive respiratory support, HFNC can rapidly improve oxygenation. At present, HFNC can be applied to patients with acute hypoxemic respiratory failure, patients after surgical operation, patients with respiratory failure without trachea cannula, patients with immunosuppression, patients with cardiac insufficiency and the like.

"NIV", i.e., non-invasine ventilation, refers to non-invasive mechanical ventilation other than tracheal intubation and tracheotomy.

"EC50", i.e., concentration for 50% of maximal effect, refers to a concentration that causes 50% of maximal effect.

"IC50" represents 50% inhibitory concentration, i.e., a concentration of drug or inhibitor required to inhibit half of a given biological process.

"TICD50" represents 50% tissue culture infective dose, and is a standard method for determining viral titer.

### DETAILED DESCRIPTION

The technical solution of the present invention will be further illustrated below through specific examples, which are not intended to limit the protection scope of the present invention. Some nonessential modifications and adjustments made by other people according to the concept of the present invention still fall within the protection scope of the present invention.

The materials, reagents, and the like used in the following examples can be commercially available , unless otherwise indicated.

### Example 1. Preparation of Anti-Spike Protein Antiboby

An amino acid sequence of the antibody is shown in Table 1, wherein a VH and a CH form a heavy chain of the antibody, and a VL and a CL form a light chain of the antibody; the CH has a sequence set forth in SEQ ID NO: 51, and the CL has a sequence set forth in SEQ ID NO: 52. The antibody can be prepared by the following method or other known methods: sequence optimization was performed according to codon usage bias of the host cell CHO, and a DNA sequence was obtained from the amino acid sequence. The optimized and synthesized sequence clones were cloned into vectors, then a large number of plasmids were extracted and subjected to transient expression: the linearized expression vectors were mixed well with CHO cells, and then the mixture was added into a 0.4 cm electroporation cuvette to perform electroporation; after the electroporation was completed, 1200 cells/well were plated into a 96-well cell culture plate, parent clones with high expression level were selected after about 2-3 weeks for cell amplification culture and expression level detection from 96-well to 24-well to 6-well to a shake flask, the clones with high expression level in the shake flask were selected for subcloning, and the subcloning amplification culture and expression identification were performed on the same parent clones; a stable monoclonal cell strain was selected according to the expression level and the stability of the cell strain, supernatant was collected after suspension culture for about 12 days, and an antibody with a purity of more than 95% was obtained by proA affinity capture and anion and cation chromatography. The VH of antibody 1 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 4 and an FR4 set forth in SEQ ID NO: 45, and the VL of antibody 1 is set forth in SEQ ID NO: 50;
the VH of antibody 2 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 7 and an FR4 set forth in SEQ ID NO: 45, and the VL of antibody 2 is set forth in SEQ ID NO: 50;
the VH of antibody 3 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 13 and an FR4 set forth in SEQ ID NO: 45, and the VL of antibody 3 is set forth in SEQ ID NO: 50;
the VH of antibody 4 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 14 and an FR4 set forth in SEQ ID NO: 45, and the VL of antibody 4 is set forth in SEQ ID NO: 50;
the VH of antibody 5 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 16 and an FR4 set forth in SEQ ID NO: 45, and the VL of antibody 5 is set forth in SEQ ID NO: 50;
the VH of antibody 6 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 17 and an FR4 set forth in SEQ ID NO: 45, and the VL of antibody 6 is set forth in SEQ ID NO: 50;
the VH of antibody 7 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 18 and an FR4 set forth in SEQ ID NO: 45, and the VL of antibody 7 is set forth in SEQ ID NO: 50;
the VH of antibody 8 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 19 and an FR4 set forth in SEQ ID NO: 45, and the VL of antibody 8 is set forth in SEQ ID NO: 50;
the VH of antibody 9 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 20 and an FR4 set forth in SEQ ID NO: 45, and the VL of antibody 9 is set forth in SEQ ID NO: 50;
the VH of antibody 10 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 21 and an FR4 set forth in SEQ ID NO: 45, and the VL of antibody 10 is set forth in SEQ ID NO: 50;
the VH of antibody 11 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 22 and an FR4 set forth in SEQ ID NO: 45, and the VL of antibody 11 is set forth in SEQ ID NO: 50;
the VH of antibody 12 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 23 and an FR4 set forth in SEQ ID NO: 45, and the VL of antibody 12 is set forth in SEQ ID NO: 50;
the VH of antibody 13 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 26 and an FR4 set forth in SEQ ID NO: 45, and the VL of antibody 13 is set forth in SEQ ID NO: 50;
the VH of antibody 14 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 27 and an FR4 set forth in SEQ ID NO: 45, and the VL of antibody 14 is set forth in SEQ ID NO: 50;
the VH of antibody 15 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 28 and an FR4 set forth in SEQ ID NO: 45, and the VL of antibody 15 is set forth in SEQ ID NO: 50;
the VH of antibody 16 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 30 and an FR4 set forth in SEQ ID NO: 45, and the VL of antibody 16 is set forth in SEQ ID NO: 50;
the VH of antibody 17 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 31 and an FR4 set forth in SEQ ID NO: 45, and the VL of antibody 17 is set forth in SEQ ID NO: 50;
the VH of antibody 18 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 32 and an FR4 set forth in SEQ ID NO: 45, and the VL of antibody 18 is set forth in SEQ ID NO: 50;
the VH of antibody 19 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 33 and an FR4 set forth in SEQ ID NO: 45, and the VL of antibody 19 is set forth in SEQ ID NO: 50;
the VH of antibody 20 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 34 and an FR4 set forth in SEQ ID NO: 45, and the VL of antibody 20 is set forth in SEQ ID NO: 50;
the VH of antibody 21 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 35 and an FR4 set forth in SEQ ID NO: 45, and the VL of antibody 21 is set forth in SEQ ID NO: 50;
the VH of antibody 22 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 37 and an FR4 set forth in SEQ ID NO: 45, and the VL of antibody 22 is set forth in SEQ ID NO: 50;
the VH of antibody 23 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 38 and an FR4 set forth in SEQ ID NO: 45, and the VL of antibody 23 is set forth in SEQ ID NO: 50;
the VH of antibody 24 comprises an FR1 set forth in SEQ ID NO: 42, a VH CDR1 set forth in SEQ ID NO: 1, an FR2 set forth in SEQ ID NO: 43, a VH CDR2 set forth in SEQ ID NO: 2, an FR3 set forth in SEQ ID NO: 44, a VH CDR3 set forth in SEQ ID NO: 105 and an FR4 set forth in SEQ ID NO: 45, and the VL of antibody 24 is set forth in SEQ ID NO: 50.

**Table 1 Amino acid sequence**

| Name | No. | Amino acid sequence |
|---|---|---|
| VH CDR1 | SEQ ID NO.1 | FTFSSYA |
| VH CDR2 | SEQ ID NO.2 | AISGSGGSTY |
| | SEQ ID NO.3 | ADYASTVYDYR |
| | SEQ ID NO.4 | DRDPSASYYLD |
| | SEQ ID NO.5 | GPLGFSAGGLP |
| | SEQ ID NO.6 | GPFGIYDGYLP |
| | SEQ ID NO.7 | GPYGYSPGYLP |
| | SEQ ID NO.8 | GPFGFSDGYLP |
| | SEQ ID NO.9 | HDDASPPGPDS |
| | SEQ ID NO.10 | GPFGYSAGYLP |
| | SEQ ID NO.11 | ADYASTVYDGR |
| | SEQ ID NO.12 | HLGPTYGGYAR |
| | SEQ ID NO.13 | GLYGYDLYLDR |
| | SEQ ID NO.14 | ETSYDGYVTLA |
| | SEQ ID NO.15 | HAEDSPPGPDS |
| VH CDR3 | SEQ ID NO.16 | GSYGYDLYLDR |
| | SEQ ID NO.17 | GPFGYSDGYLP |
| | SEQ ID NO.18 | DTGYFYATTLD |
| | SEQ ID NO.19 | DLDPDAFYYLD |
| | SEQ ID NO.20 | DRDPSAPYYLD |
| | SEQ ID NO.21 | DRDPAAYYYLD |
| | SEQ ID NO.22 | DRDPTAVYYLD |
| | SEQ ID NO.23 | ESSYDGYVDLA |
| | SEQ ID NO.24 | DRDPSAYYYLD |
| | SEQ ID NO.25 | GPFGVSDGYLP |
| | SEQ ID NO.26 | HPDESPPLPDR |
| | SEQ ID NO.27 | GPYGYSAGYLP |
| | SEQ ID NO.28 | HPTRYPAYTDP |
| | SEQ ID NO.29 | GPFGYSSGYLP |
| | SEQ ID NO.30 | ELYPGRFFPLY |
| | SEQ ID NO.31 | HRYPRSYSPLS |
| | SEQ ID NO.32 | DRDPSATYYLD |
| | SEQ ID NO.33 | DTAYYYYTTLD |
| | SEQ ID NO.34 | RVDLGLYYLDA |
| | SEQ ID NO.35 | DTRYYYLSTLD |
| | SEQ ID NO.36 | VPTRGIGRYDFL |
| | SEQ ID NO.37 | DRARYNSYLVH |
| | SEQ ID NO.38 | GPFGSSPGYLP |
| | SEQ ID NO.105 | GPFGIDDAYLP |
| VL CDR1 | SEQ ID NO.39 | QGISSY |
| VL CDR2 | SEQ ID NO.40 | AAS |
| VL CDR3 | SEQ ID NO.41 | QQHYTTP |
| Heavy chain FR1 | SEQ ID NO.42 | EVQLVESGGGLVQPGGSLRLSCTASG |
| Heavy chain FR2 | SEQ ID NO.43 | MSWVRQAPGKGLEWVS |
| Heavy chain FR3 | SEQ ID NO.44 | YADSVKGRFTISRDNSKNTLYLQMNSLRAEDTAVYYCAR |
| Heavy chain FR4 | SEQ ID NO.45 | DYWGQGTLVTVSS |
| | SEQ ID NO.46 | EVQLVESGGGLVQPGGSLRLSCTASGFTFSSYAMSWVRQAP GKGLEWVSAISGSGGSTYYADSVKGRFTISRDNSKNTLYLQ MNSLRAEDTAVYYCARDRDPSASYYLDDYWGQGTLVTVSS |
| | SEQ ID NO.47 | EVQLVESGGGLVQPGGSLRLSCTASGFTFSSYAMSWVRQAP GKGLEWVSAISGSGGSTYYADSVKGRFTISRDNSKNTLYLQ MNSLRAEDTAVYYCARGPFGYSAGYLPDYWGQGTLVTVSS |
| VH | SEQ ID NO.48 | EVQLVESGGGLVQPGGSLRLSCTASGFTFSSYAMSWVRQAP GKGLEWVSAISGSGGSTYYADSVKGRFTISRDNSKNTLYLQ MNSLRAEDTAVYYCARHLGPTYGGYARDYWGQGTLVTVSS |
| | SEQ ID NO.49 | EVQLVESGGGLVQPGGSLRLSCTASGFTFSSYAMSWVRQAP GKGLEWVSAISGSGGSTYYADSVKGRFTISRDNSKNTLYLQ MNSLRAEDTAVYYCARETSYDGYVTLADYWGQGTLVTVSS |
| | SEQ ID NO.107 | EVQLVESGGGLVQPGGSLRLSCTASGFTFSSYAMSWVRQAP GKGLEWVSAISGSGGSTYYADSVKGRFTISRDNSKNTLYLQ MNSLRAEDTAVYYCARGPFGIDDAYLPDYWGQGTLVTVSS |
| VL | SEQ ID NO.50 | DIQMTQSPSSLSASVGDRVTITCRASQGISSYLAWYQQKPGK APKLLIYAASSLQSGVPSRFSGSGSGTDFTLTISSLQPEDFATY YCQQHYTTPPTFGQGTKVEIK |
| Heavy chain constant region | SEQ ID NO.51 | ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKVEPKSCDKTHTCPPCPAPELLGGPSVFLFP PKPKDTLYITREPEVTCVVVDVSHEDPEVKFNWYVDGVEVH NAKTKPREEQYASTYRVVSVLTVLHQDWLNGKEYKCKVSN KALPAPIEKTISKAKGQPREPQVYTLPPSRDELTKNQVSLTCL VKGFYPSDIAVEWESNGQPENNYKTTPPVLDSDGSFFLYSKL TVDKSRWQQGNVFSCSVMHEALHNHYTQKSLSLSPGK |

| Light chain constant region | SEQ ID NO.52 | |
|---|---|---|
| | SEQ ID NO.53 | EVQLVESGGGLVQPGGSLRLSCTASGFTFSSYAMSWVRQAP GKGLEWVSAISGSGGSTYYADSVKGRFTISRDNSKNTLYLQ MNSLRAEDTAVYYCARDRDPSASYYLDDYWGQGTLVTVSS ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKVEPKSCDKTHTCPPCPAPELLGGPSVFLFP PKPKDTLYITREPEVTCVVVDVSHEDPEVKFNWYVDGVEVH NAKTKPREEQYASTYRVVSVLTVLHQDWLNGKEYKCKVSN KALPAPIEKTISKAKGQPREPQVYTLPPSRDELTKNQVSLTCL VKGFYPSDIAVEWESNGQPENNYKTTPPVLDSDGSFFLYSKL TVDKSRWQQGNVFSCSVMHEALHNHYTQKSLSLSPGK |
| Heavy chain | SEQ ID NO.54 | EVQLVESGGGLVQPGGSLRLSCTASGFTFSSYAMSWVRQAP GKGLEWVSAISGSGGSTYYADSVKGRFTISRDNSKNTLYLQ MNSLRAEDTAVYYCARGPFGYSAGYLPDYWGQGTLVTVSSA STK GPSVFPLAPSSKSTSGGTAALGCLVKDYFPEPVTVSWNSGAL TSGVHTFPAVLQSSGLYSLSSVVTVPSSSLGTQTYICNVNHKP SNTKVDKKVEPKSCDKTHTCPPCPAPELLGGPSVFLFPPKPK DTLYITREPEVTCVVVDVSHEDPEVKFNWYVDGVEVHNAKT KPREEQYASTYRVVSVLTVLHQDWLNGKEYKCKVSNKALP APIEKTISKAKGQPREPQVYTLPPSRDELTKNQVSLTCLVKGF YPSDIAVEWESNGQPENNYKTTPPVLDSDGSFFLYSKLTVDK SRWQQGNVFSCSVMHEALHNHYTQKSLSLSPGK |
| | SEQ ID NO.55 | EVQLVESGGGLVQPGGSLRLSCTASGFTFSSYAMSWVRQAP GKGLEWVSAISGSGGSTYYADSVKGRFTISRDNSKNTLYLQ MNSLRAEDTAVYYCARHLGPTYGGYARDYWGQGTLVTVSS ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKVEPKSCDKTHTCPPCPAPELLGGPSVFLFP PKPKDTLYITREPEVTCVVVDVSHEDPEVKFNWYVDGVEVH NAKTKPREEQYASTYRVVSVLTVLHQDWLNGKEYKCKVSN KALPAPIEKTISKAKGQPREPQVYTLPPSRDELTKNQVSLTCL VKGFYPSDIAVEWESNGQPENNYKTTPPVLDSDGSFFLYSKL TVDKSRWQQGNVFSCSVMHEALHNHYTQKSLSLSPGK |
| | SEQ ID NO.56 | EVQLVESGGGLVQPGGSLRLSCTASGFTFSSYAMSWVRQAP GKGLEWVSAISGSGGSTYYADSVKGRFTISRDNSKNTLYLQ MNSLRAEDTAVYYCARETSYDGYVTLADYWGQGTLVTVSS ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKVEPKSCDKTHTCPPCPAPELLGGPSVFLFP PKPKDTLYITREPEVTCVVVDVSHEDPEVKFNWYVDGVEVH NAKTKPREEQYASTYRVVSVLTVLHQDWLNGKEYKCKVSN KALPAPIEKTISKAKGQPREPQVYTLPPSRDELTKNQVSLTCL VKGFYPSDIAVEWESNGQPENNYKTTPPVLDSDGSFFLYSKL TVDKSRWQQGNVFSCSVMHEALHNHYTQKSLSLSPGK |
| | SEQ ID NO.108 | EVQLVESGGGLVQPGGSLRLSCTASGFTFSSYAMSWVRQAP GKGLEWVSAISGSGGSTYYADSVKGRFTISRDNSKNTLYLQ MNSLRAEDTAVYYCARGPFGIDDAYLPDYWGQGTLVTVSS ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKVEPKSCDKTHTCPPCPAPELLGGPSVFLFP PKPKDTLYITREPEVTCVVVDVSHEDPEVKFNWYVDGVEVH NAKTKPREEQYASTYRVVSVLTVLHQDWLNGKEYKCKVSN KALPAPIEKTISKAKGQPREPQVYTLPPSRDELTKNQVSLTCL VKGFYPSDIAVEWESNGQPENNYKTTPPVLDSDGSFFLYSKL TVDKSRWQQGNVFSCSVMHEALHNHYTQKSLSLSPGK |
| Light chain | SEQ ID NO.57 | DIQMTQSPSSLSASVGDRVTITCRASQGISSYLAWYQQKPGK APKLLIYAASSLQSGVPSRFSGSGSGTDFTLTISSLQPEDFATY YCQQHYTTPPTFGQGTKVEIKRTVAAPSVFIFPPSDEQLKSGT ASVVCLLNNFYPREAKVQWKVDNALQSGNSQESVTEQDSK DSTYSLSSTLTLSKADYEKHKVYACEVTHQGLSSPVTKSFNR GEC |
| 6-HB interference polypeptide | SEQ ID NO.58 | DISGINASVVNIQKEIDRLNEVAKNLNESLIDLQEL |
| Linker | SEQ ID NO.59 | GGGGSGGGGSGGGGSGGGGS |

**Table 2 Amino acid sequence**

| Name | No. | Amino acid sequence |
|---|---|---|
| VH CDR1 | SEQ ID NO.60 | SYAMS |
| VH CDR2 | SEQ ID NO.61 | AISGSGGSTYYADSVKG |
| | SEQ ID NO.62 | ADYASTVYDYRDY |
| | SEQ ID NO.63 | DRDPSASYYLDDY |
| | SEQ ID NO.64 | GPLGFSAGGLPDY |
| | SEQ ID NO.65 | GPFGIYDGYLPDY |
| | SEQ ID NO.66 | GPYGYSPGYLPDY |
| | SEQ ID NO.67 | GPFGFSDGYLPDY |
| | SEQ ID NO.68 | HDDASPPGPDSDY |
| VH CDR3 | SEQ ID NO.69 | GPFGYSAGYLPDY |
| | SEQ ID NO.70 | ADYASTVYDGRDY |
| | SEQ ID NO.71 | HLGPTYGGYARDY |
| | SEQ ID NO.72 | GLYGYDLYLDRDY |
| | SEQ ID NO.73 | ETSYDGYVTLADY |
| | SEQ ID NO.74 | HAEDSPPGPDSDY |
| | SEQ ID NO.75 | GSYGYDLYLDRDY |
| | SEQ ID NO.76 | GPFGYSDGYLPDY |
| | SEQ ID NO.77 | DTGYFYATTLDDY |
| | SEQ ID NO.78 | DLDPDAFYYLDDY |
| | SEQ ID NO.79 | DRDPSAPYYLDDY |
| | SEQ ID NO.80 | DRDPAAYYYLDDY |
| | SEQ ID NO.81 | DRDPTAVYYLDDY |
| | SEQ ID NO.82 | ESSYDGYVDLADY |
| | SEQ ID NO.83 | DRDPSAYYYLDDY |
| | SEQ ID NO.84 | GPFGVSDGYLPDY |
| | SEQ ID NO.85 | HPDESPPLPDRDY |
| | SEQ ID NO.86 | GPYGYSAGYLPDY |
| | SEQ ID NO.87 | HPTRYPAYTDPDY |
| | SEQ ID NO.88 | GPFGYSSGYLPDY |
| | SEQ ID NO.89 | ELYPGRFFPLYDY |
| | SEQ ID NO.90 | HRYPRSYSPLSDY |
| | SEQ ID NO.91 | DRDPSATYYLDDY |
| | SEQ ID NO.92 | DTAYYYYTTLDDY |
| | SEQ ID NO.93 | RVDLGLYYLDADY |
| | SEQ ID NO.94 | DTRYYYLSTLDDY |
| | SEQ ID NO.95 | VPTRGIGRYDFLDY |
| | SEQ ID NO.96 | DRARYNSYLVHDY |
| | SEQ ID NO.97 | GPFGSSPGYLPDY |
| | SEQ ID NO.106 | GPFGIDDAYLPDY |
| VL CDR1 | SEQ ID NO.98 | RASQGISSYLA |
| VL CDR2 | SEQ ID NO.99 | AASSLQS |
| VL CDR3 | SEQ ID NO.100 | QQHYTTPPT |
| Heavy chain FR1 | SEQ ID NO.101 | EVQLVESGGGLVQPGGSLRLSCTASGFTFS |
| Heavy chain FR2 | SEQ ID NO.102 | WVRQAPGKGLEWVS |
| Heavy chain FR3 | SEQ ID NO.103 | RFTISRDNSKNTLYLQMNSLRAEDTAVYYCAR |
| Heavy chain FR4 | SEQ ID NO.104 | WGQGTLVTVSS |

### Example 2. Preparation of Fusion Protein

A fusion protein can be prepared by the following method or other known methods according to the sequence of the fusion protein described herein. The fusion protein comprises a first polypeptide and a third polypeptide which have the same sequence, and a second polypeptide and a fourth polypeptide which have the same sequence; the first polypeptide comprises a heavy chain (or a heavy chain fragment), a linker, and a 6-HB interference polypeptide, and the second polypeptide comprises a light chain. Sequence optimization was performed according to codon usage bias of the host cell CHO, and a DNA sequence was obtained from the amino acid sequence. The fusion protein A was taken as an example, an amino acid sequence and a nucleic acid sequence of the first polypeptide of the fusion protein A are set forth in SEQ ID NO: 111 and SEQ ID NO: 109, respectively, an amino acid sequence and a nucleic acid sequence of the second polypeptide of the fusion protein A are set forth in SEQ ID NO: 112 and SEQ ID NO: 110, respectively. The optimized and synthesized sequence clones were cloned into vectors, then a large number of plasmids were extracted and subjected to transient expression: the linearized expression vectors were mixed well with CHO cells, and then the mixture was added into a 0.4 cm electroporation cuvette to perform electroporation; after the electroporation was completed, 1200 cells/well were plated into a 96-well cell culture plate, parent clones with high expression level were selected after about 2-3 weeks for cell amplification culture and expression level detection from 96-well to 24-well to 6-well to a shake flask, the clones with high expression level in the shake flask were selected for subcloning, and the subcloning amplification culture and expression identification were performed on the same parent clones; a stable monoclonal cell strain was selected according to the expression level and the stability of the cell strain, supernatant was collected after suspension culture for about 12 days, and an antibody with a purity of more than 95% was obtained by proA affinity capture and anion and cation chromatography. The sequence of the fusion protein A was verified by sequencing as described above.

**Table 3. Nucleic acid sequence and amino acid sequence of fusion protein A**

| Name | No. | Sequence |
|---|---|---|
| Nucleic acid sequence of a first polypeptide | 109 | GAAGTGCAATTGGTGGAAAGCGGCGGCCTGGTGCAGCCGGGCGGCAG CCTGCGCCTGAGCTGCGCGGCGTCCGGATTTACCTTTAGCAGCTATGCGATG AGCTGGGTGCGCCAGGCACCGGGCAAAGGCCTCGAATGGGTGAGCGCGAT TAGCGGCAGCGGCGGCAGCACCTATTATGCGGATAGCGTGAAAGGCCGCTT TACCATTAGCCGCGATAATTCGAAAAACACCCTGTATCTGCAAATGAACAG CCTGCGTGCGGAAGATACTGCAGTGTATTATTGCGCGCGTGGTCCGTTCGGT ATCGACGACGCTTACCTGCCGGATTATTGGGGCCAAGGCACCCTGGTGACG GTGAGCAGCGCCTCTACCAAGGGCCCCTCTGTGTTTCCTCTGGCTCCCTCCA GCAAGTCTACCTCTGGTGGAACAGCTGCCCTGGGCTGCCTGGTCAAGGATT ACTTTCCTGAGCCTGTCACCGTGTCCTGGAACTCTGGCGCTCTGACATCTGG CGTGCACACCTTTCCAGCTGTGCTCCAGTCCTCCGGCCTGTACTCTCTGTCCT CTGTCGTGACCGTGCCTTCTAGCTCTCTGGGCACCCAGACCTACATCTGCAA TGTGAACCACAAGCCTTCCAACACCAAGGTGGACAAGAAGGTGGAACCCA AGTCCTGCGACAAGACCCACACCTGTCCTCCATGTCCTGCTCCAGAACTGCT CGGCGGACCTTCCGTGTTCCTGTTTCCTCCAAAGCCTAAGGACACCCTGATG |
| | | ATCTCTCGGACCCCTGAAGTGACCTGCGTGGTGGTGGATGTGTCTCACGAA GATCCCGAAGTGAAGTTCAACTGGTACGTGGACGGCGTGGAAGTGCACAAC GCCAAGACCAAGCCTAGAGAGGAACAGTACAACTCCACCTACAGAGTGGT GTCCGTGCTGACCGTGCTGCACCAGGATTGGCTGAACGGCAAAGAGTACAA GTGCAAGGTGTCCAACAAGGCCCTGCCTGCTCCTATCGAAAAGACCATCTC CAAGGCCAAGGGCCAGCCTAGGGAACCCCAGGTTTACACCTTGCCTCCATC TCGGGACGAGCTGACCAAGAACCAGGTGTCCCTGACCTGTCTCGTGAAGGG CTTCTACCCCTCCGACATCGCCGTGGAATGGGAGTCTAATGGCCAGCCTGA GAACAACTACAAGACAACCCCTCCTGTGCTGGACTCCGACGGCTCATTCTTC CTGTACTCCAAGCTGACAGTGGACAAGTCCAGATGGCAGCAGGGCAACGTG TTCTCCTGCTCCGTGATGCACGAGGCCCTGCACAATCACTACACCCAGAAGT CCCTGTCTCTGTCCCCTGGAGGAGGAGGCTCCGGAGGAGGCGGAAGCGGAG GAGGAGGATCTGGCGGCGGCGGATCTGACATCTCTGGCATCAATGCCTCTG TGGTGAACATCCAGAAGGAGATTGACAGACTGAATGAGGTGGCTAAGAAC CTGAATGAGTCCCTGATTGACCTCCAAGAACTG |
| Nucleic acid sequence of a second polypeptide | 110 | GATATCCAGATGACCCAGTCCCCCAGCTCCCTGAGCGCTAGCGTGGGCGAC CGGGTGACCATCACCTGCAGGGCCTCCCAGGGCATCAGCTCCTACCTGGCT TGGTATCAGCAGAAGCCTGGCAAGGCCCCTAAGCTGCTGATCTATGCCGCT TCCTCCCTGCAGTCCGGCGTGCCTTCCAGGTTCAGCGGCTCCGGCAGCGGCA CCGACTTCACCCTGACCATCTCCTCCCTGCAGCCTGAGGACTTCGCCACCTA TTATTGCCAGCAGCACTATACCACCCCTCCTACCTTTGGCCAGGGCACCAAG GTGGAGATCAAGCGTACGGTGGCTGCACCATCTGTCTTCATCTTCCCGCCAT CTGATGAGCAGTTGAAATCTGGAACTGCCTCTGTTGTGTGCCTGCTGAATAA CTTCTATCCCAGAGAGGCCAAAGTACAGTGGAAGGTGGATAACGCCCTCCA ATCGGGTAACTCCCAGGAGAGTGTCACAGAGCAGGACAGCAAGGACAGCA CCTACAGCCTCAGCAGCACCCTGACGCTGAGCAAAGCAGACTACGAGAAAC ACAAAGTCTACGCCTGCGAAGTCACCCATCAGGGCCTGAGCTCGCCCGTCA CAAAGAGCTTCAACAGGGGAGAGTGT |
| Amino acid sequence of a first polypeptide | 111 | EVQLVESGGGLVQPGGSLRLSCTASGFTFSSYAMSWVRQAPGKGLEWVSAISG SGGSTYYADSVKGRFTISRDNSKNTLYLQMNSLRAEDTAVYYCARGPFGIDDA YLPDYWGQGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSNT KVDKKVEPKSCDKTHTCPPCPAPELLGGPSVFLFPPKPKDTLYITREPEVTCVVV DVSHEDPEVKFNWYVDGVEVHNAKTKPREEQYASTYRVVSVLTVLHQDWLN GKEYKCKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQQGNV FSCSVMHEALHNHYTQKSLSLSPGGGGSGGGGSGGGGSGGGGSDISGINASVV NIQKEIDRLNEVAKNLNESLIDLQEL |
| Amino acid sequence of a second polypeptide | 112 | DIQMTQSPSSLSASVGDRVTITCRASQGISSYLAWYQQKPGKAPKLLIYAASSL QSGVPSRFSGSGSGTDFTLTISSLQPEDFATYYCQQHYTTPPTFGQGTKVEIKRT VAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC |

### Example 3. Assay of Binding Activity of Antibodies to Spike Protein of SARS-CoV-2

ELISA assay was performed on the above antibody, and the assay method was as follows: a 96-well plate (Corning, 9018) was coated with spike-RBD-mFC (sino biologicals), sealed with tape and stored; the plate was washed 3 times in a washing buffer PBST (PBS, 0.05% Tween 20), and then blocking solution (10 mg/mL BSA at 200 µL/well, the solvent was the washing buffer) was added; after incubation (1 h, 37 °C), the plate was washed 3 times with the washing buffer, and then 100 µL of gradient-diluted samples were added to each well; after incubation (1.5 h, 37 °C), the plate was washed with the washing buffer, followed by the addition of anti-human κ light chain antibody-peroxidase conjugate (diluted to 1:2000 in blocking solution, 100 µL/well); the plate was washed with the washing buffer and the test samples were incubated (1 h, 37 °C) before adding 100 µL of TMB (Tetramethylbenzidine, Biopanda TMB-S-001) substrate/well; after 10 min of substrate development, the reaction was terminated by adding 100 µL/well of 0.1 M H₂SO₄, and then the absorbance of the 96-well plate at 450 nm was measured.

EC50 was calculated from the absorbance values, and the EC50 values for the binding of various antibodies to the spike protein in SARS-CoV-2 are shown in Table 4.

**Table 4 EC50 values for the binding of various antibodies to the spike protein in SARS-CoV-2**

| | | | | | | | | | |
|---|---|---|---|---|---|---|---|---|---|
| Antibody | 1 | 2 | 3 | 4 | 5 | 6 | 7 | 8 | 9 |
| EC50 (nM) | 0.158 | 0.352 | 0.352 | 0.155 | 0.001 | 0.624 | 0.063 | 0.378 | 2.76 |
| Antibody | 10 | 11 | 12 | 13 | 14 | 24 | 16 | 17 | 18 |
| EC50 (nM) | 1.264 | 1.128 | 0.342 | 0.004 | 0.47 | 0.348 | 0.115 | 0.022 | 0.144 |
| Antibody | 19 | 20 | 21 | 22 | 23 | / | | | |
| EC50 (nM) | 0.084 | 0.065 | 0.071 | 0.167 | 0.347 | | | | |

### Example 4. Assay of Binding Activity of Fusion Protein A to Spike Protein in SARS-CoV-2 and Mutant

1) ELISA assay was as follows: 1 µg/mL spike-RBD at 100 µL/well was added to a 96-well plate, and the plate was placed overnight at 4 °C; the plate was washed twice with 300 µL/well PBST; the plate was blocked with 300 µL/well blocking buffer (3% BSA in PBST) and incubated at 37 °C for 2 h; 10 fusion protein A diluted 3-fold were added sequentially to the blocked 96-well plate with the highest concentration of 5 µg/mL, the plate was incubated at 37 °C for 1 h, and two duplicate wells were repeated for each concentration; the plate was added with 1:10,000 diluted HRP-goat anti-human IgG-Fc antibody (Abcam, Ab97225) at 100µL/well and incubated at 37 °C for 1 h; the plate was washed 8 times with 300 µL/well PBST; the plate was added with 100 µL/well TMB and incubated at 37 °C for 15-25 min; the reaction was terminated with 50 µL/well of 0.2 M H₂SO₄ and reading was performed within 30 min. Spike-RBD was classified into wild-type Spike S1 RBD (Acro Biosystems, SPD-C52H3), variant S protein RBD (V367F) (Acrosystems, SPD-S52H4), variant S protein RBD (N354D, D364Y) (Acrosystems, SPD-S52H3), variant S protein RBD(W436R)(Acrosystems, SPD-S52H7), and variant S1 protein (D614G) (Novoprotein, DRA57).

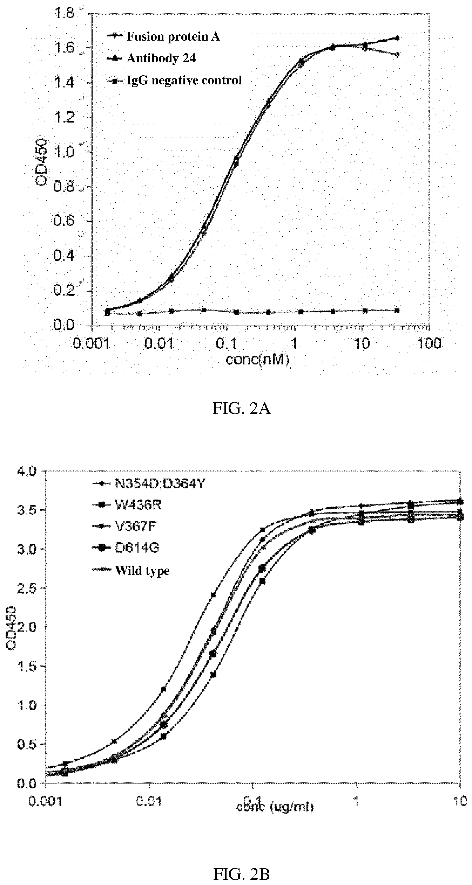

As shown in FIG. 2A and FIG. 2B, fusion protein A bound to wild-type Spike S1 RBD with high affinity. Fusion protein A bound to wild-type Spike S1 RBD and variants thereof with high affinity: the EC50 value of fusion protein A binding to wild-type Spike S1 RBD was 0.15 nM; the binding activity of the fusion protein A to the Spike S1 RBD variant (N354D/D364Y; V37F; W436R) was similar to that of the wild-type Spike S1 RBD. The fusion protein A can bind to wild-type virus and virus mutants with high affinity.

2) Biacore (biomolecular interaction analysis): the fusion protein A was coated on the protein A chip at a concentration of 10 µg/mL; binding kinetics were measured using Biacore at 25 °C; the chip was equilibrated; recombinant Spike S1 RBD protein or spike trimer (Acrobiosystems, SPN-C52H8) was passed through the fusion protein A-coated chip at a concentration of 10-400 nM; background and non-specific binding signals were subtracted by flow-through buffer only (0.05% Tween-20 in PBS) as a control; kinetic constants were calculated using the 1:1 Langmuir binding model on Biacore data analysis software.

An EC50 value of the binding of the fusion protein A to wild-type Spike S1 RBD was 4.54 nM, and an EC50 value of the binding of the fusion protein A to natural spike trimer was 0.773 nM according to evaluation using Biacore.

### Example 5. Activity Assay of Antibody or Fusion Protein A Blocking the Binding of Spike Protein in SARS-CoV-2 to Angiotensin Converting Enzyme 2

1) The ability of some of the antibodies described above to block the binding of spike protein to angiotensin converting enzyme 2 (ACE2) was detected by competitive ELISA. The detection method was as follows: a 96-well plate (Corning, 9018) was coated with spike-RBD-mFC (sino biologicals), seal with tape and stored at 4 °C overnight; the plate was washed 3 times in washing buffer (PBS, 0.05% Tween 20) before the addition of blocking solution (10 mg/mL BSA at 200 µL/well, the solvent was the washing buffer); after incubation (2 h, 37 °C), the plate was washed 3 times in washing buffer, antibody or fusion protein samples at different concentrations were added, and then biotinylated angiotensin-converting enzyme 2 (50 ng/mL) was added; after incubation (1 h, 37 °C), the plate was washed 3 times with the washing buffer, then 100 µL of streptavidin-peroxidase conjugate (1:10,000 diluted in a blocking solution) was added to each well; after incubation (1.0 h, 37 °C), the plate was washed with the washing buffer, and added with 100 µL/well of TMB substrate; after color development for 10 min, the reaction was terminated by adding 50 µL/well of 0.1 M H₂SO₄, and then the absorbance of the plate at 450 nm was measured.

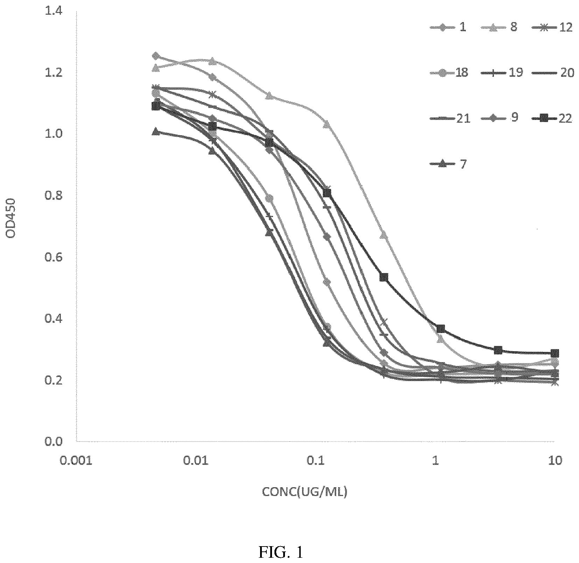

The curves of the various antibodies described above blocking the binding of the spike protein of COVID-19 to ACE2 are shown in FIG. 1.

2) The activity of the fusion protein A blocking the binding of the spike protein in SARS-CoV-2 to angiotensin converting enzyme 2 could be determined by the same or similar method.

The ability of the fusion protein A described above to block the binding of spike protein to ACE2was detected by competitive ELISA. The detection method was as follows: a 96-well plate (Costar, 9018) was coated with 1 µg/mL Spike S1 RBD (Aero Biosystems, SPD-C82E9) at 100 µL/well, sealed with tape and stored at 4 °C overnight; the plate was washed 3 times with the washing buffer PBST (PBS containing 0.05% Tween 20) at 300 µL/well, and then the plate was added with 300 µL/well blocking solution (PBST containing 3% BSA); after incubation (2 h, 37 °C), 9 fusion protein A diluted 3-fold were added sequentially to the blocked 96-well plate with the highest concentration of 7.5 µg/mL, the plate was incubated at 37 °C for 1 h, and two duplicate wells were repeated for each concentration; then the plate was added with 25 ng/mL biotinylated ACE2 (soluble ACE2, Acrobiosystems, AC2-H5257) at 100 µL/well; after incubation (1 h, 37 °C), the plate was washed 8 times with 300 µL/well PBST; then 100 µL of streptavidin-peroxidase conjugate (10,000-fold diluted in a blocking solution) was added to each well (Jackson, 016-030-084); the plate was washed with 8 times with 300 µL/well PBST; the plate was added with 100 µL/well TMB and incubated at 37 °C for 15-25 min; the reaction was terminated with 50 µL/well of 0.2 M H₂SO4 and then the absorbance of the plate at 450 nm was measured.

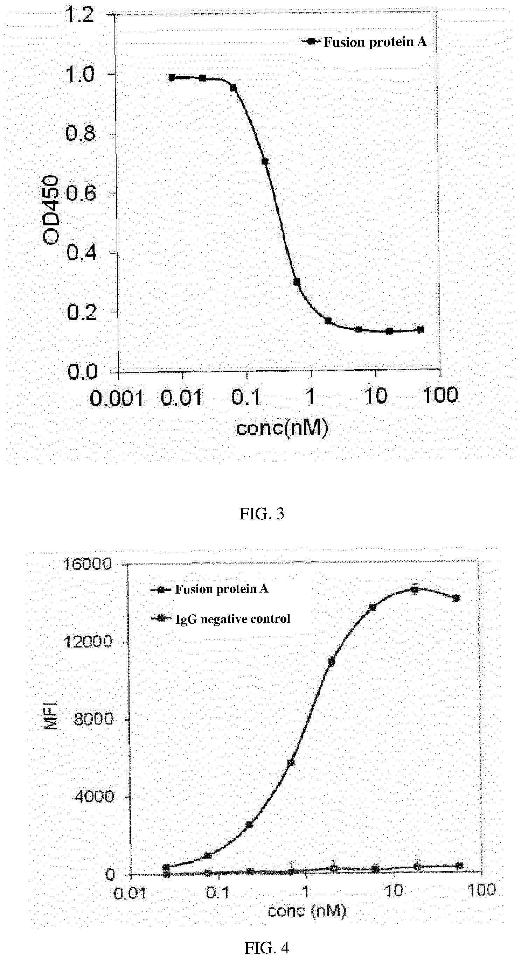

As shown in FIG. 3, the fusion protein A could bind to Spike S1 RBD and effectively blocked the binding of RBD to ACE2.

### Example 6. Binding Assay of Fusion Protein A to Spike Protein on Cells

Cells were collected by centrifugation and washed with 1× flow buffer (PBS containing 0.1% sodium azide and 5% FBS) (cells were CHO cells expressing SARS-CoV-2 spike or negative cells); sample preparation: the fusion protein A was 3-fold diluted with the initial concentration of 8.33 µg/mL and 8 diluted concentrations in total; only one concentration of 100 nM was used to detect spike-negative cells(i.e., negative cells did not express spike protein); the diluted fusion protein A was added into cells, and the cells were incubated for 1 h on ice at a concentration of 100 µL/well; the plate was washed with flow buffer for 3 times at 300 µL/well; the plate was added with the diluted PE-goat anti-human IgG-Fc antibody (Thermo Fisher, 12-4998-82) at 100 µL/well and incubated on ice for 1 h; the plate was washed with flow buffer for 3 times at 300 µL/well; the cells were resuspended in flow buffer at 200 µL/well; analysis was performed on CytoFlex and data was analyzed using flowjo10.1.

CHO cells expressing SARS-CoV-2 spike: the expression vector pCMV3-untagged (Sino Biological Inc., VG40589) containing the full-length gene of spike protein was used to transfect CHO-K1 cells by electrotransfection, followed by pressure selection and flow sorting of hygromycin (using the label of biotinylated ACE2 and the label of SA-PE (streptavidin labeled with phycoerythrin)) to screen CHO cells expressing SARS-CoV-2 spike.

As shown in FIG. 4, the fusion protein A could bind to the CHO cell strain over-expressing spike with high affinity, and the EC50 value was 0.5 nM. In addition, the assay also verified that fusion protein A did not bind to cells that did not express spike protein (e.g., CHO cells, Raji cells, Jurkat cells, TF1 cells, H929 cells, and RPMI8226). This indicates that the binding of the fusion protein to the spike protein in SARS-CoV-2 is specific and should not bind non-specifically to spike negative cells, and therefore should not produce off-target effects *in vivo.*

### Example 7. Fusion Protein A for Neutralization of Pseudo viruses

The assay evaluated the ability of the fusion protein A to inhibit spike-pseudotyped pseudovirus from infecting cells expressing ACE2 *in vitro.* Vero cells were adopted to detect the ability of the fusion protein A to inhibit SARS-CoV-2 pseudovirus with luciferase gene from infecting cells. The main principle was as follows: an African green monkey kidney cell line Vero expressing an ACE2 receptor similar to a human was adopted as a susceptible cell; the fusion protein A with different concentrations was incubated with a SARS-CoV-2-Fluc pseudovirus system; after the fusion protein A was incubated and combined with the pseudovirus, the virus was blocked to infect into the Vero cell; the pseudovirus could not effectively infect the cell, and the luciferase reporter gene on the genome of the pseudovirus could not be expressed in the cell and generated a fluorescent signal; since the signal value of the fluorescence signal was in negative correlation with the concentration of the added fusion protein A, the ability of the fusion protein A *in vitro* to inhibit virus infection could be detected.

The assay method was as follows: on the first day of infection, the fusion protein A was 2-fold diluted in a 96-well plate, an equal volume of pseudovirus stock solution Pseudovirus stock (2 × 10⁴, TICD₅₀) was added to the diluted fusion protein A, the mixture was mixed well and incubated at 37 °C for 1 h, the negative control wells were not added with pseudovirus stock (Beijing Tiantan Pharmaceutical & Biological Technology Development Co. Ltd., A01007424), and the positive control wells were added with virus but not fusion protein A; during the incubation, Vero cells were harvested by trypsinization (the Cell Bank of the Chinese Academy of Sciences, GN017) and washed once with PBS, and then 50 µL of DMEM medium containing 2 × 10⁴ cells was added to the incubated fusion protein A-pseudovirus mixture; the mixture was incubated in a 37 °C incubator for 24-28 hours, and added with 100 µL of luciferase substrate (Novizan, DD1201); the reading was performed after two minutes. Inhibition rate was calculated by the formula: inhibition% = (1 - (sample group-negative control group) / (positive control group - negative control group) × 100.

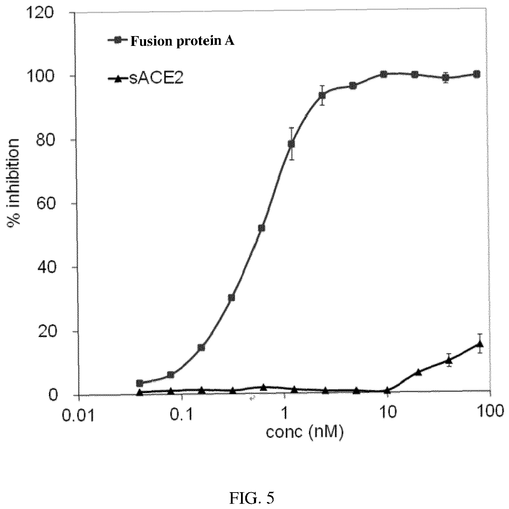

As shown in FIG. 5, the inhibitory ability exhibited by soluble ACE2 at the same concentration was very limited, and fusion protein A effectively inhibited SARS-CoV-2 spike-pseudovirus infection into Vero cells with an IC50 value of 0.5 nM.

## Claims

1. An antibody or an antigen-binding fragment, wherein the antibody or the antigen-binding fragment specifically binds to a spike protein of SARS-CoV-2 or SARS-CoV, and the antibody or the antigen-binding fragment at least comprises one or more of a VH CDR1 set forth in SEQ ID NO: 1, a VH CDR2 set forth in SEQ ID NO: 2, a VH CDR3 set forth in any one of SEQ ID NOs: 3-38 or 105, a VL CDR1 set forth in SEQ ID NO: 39, a VL CDR2 set forth in SEQ ID NO: 40, and a VL CDR3 set forth in SEQ ID NO: 41.

2. The antibody or the antigen-binding fragment according to claim 1, wherein the antibody or the antigen-binding fragment at least comprises the VH CDR1 set forth in SEQ ID NO: 1, the VH CDR2 set forth in SEQ ID NO: 2, the VH CDR3 set forth in any one of SEQ ID NOs: 3-38 or 105, the VL CDR1 set forth in SEQ ID NO: 39, the VL CDR2 set forth in SEQ ID NO: 40, and the VL CDR3 set forth in SEQ ID NO: 41.

3. The antibody or the antigen-binding fragment according to claim 2, wherein the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 4, the VL CDR1 set forth in SEQ ID NO: 39, the VL CDR2 set forth in SEQ ID NO: 40, and the VL CDR3 set forth in SEQ ID NO: 41; or
the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 10, the VL CDR1 set forth in SEQ ID NO: 39, the VL CDR2 set forth in SEQ ID NO: 40, and the VL CDR3 set forth in SEQ ID NO: 41; or
the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 12, the VL CDR1 set forth in SEQ ID NO: 39, the VL CDR2 set forth in SEQ ID NO: 40, and the VL CDR3 set forth in SEQ ID NO: 41; or
the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 14, the VL CDR1 set forth in SEQ ID NO: 39, the VL CDR2 set forth in SEQ ID NO: 40, and the VL CDR3 set forth in SEQ ID NO: 41; or
the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 105, the VL CDR1 set forth in SEQ ID NO: 39, the VL CDR2 set forth in SEQ ID NO: 40, and the VL CDR3 set forth in SEQ ID NO: 41.

4. The antibody or the antigen-binding fragment according to any one of claims 1-3, wherein framework regions of a heavy chain variable region of the antibody or the antigen-binding fragment comprise a heavy chain FR1, a heavy chain FR2, a heavy chain FR3 and a heavy chain FR4, and the heavy chain FR1 of the antibody or the antigen-binding fragment comprises a sequence set forth in SEQ ID NO: 42, a sequence having at least 90% identity to the sequence set forth in SEQ ID NO: 42, or an amino acid sequence having one or more conservative amino acid substitutions as compared to the sequence set forth in SEQ ID NO: 42; and/or
the heavy chain FR2 of the antibody or the antigen-binding fragment comprises a sequence set forth in SEQ ID NO: 43, a sequence having at least 90% identity to the sequence set forth in SEQ ID NO: 43, or an amino acid sequence having one or more conservative amino acid substitutions as compared to the sequence set forth in SEQ ID NO: 43; and/or
the heavy chain FR3 of the antibody or the antigen-binding fragment comprises a sequence set forth in SEQ ID NO: 44, a sequence having at least 90% identity to the sequence set forth in SEQ ID NO: 44, or an amino acid sequence having one or more conservative amino acid substitutions as compared to the sequence set forth in SEQ ID NO: 44; and/or
the heavy chain FR4 of the antibody or the antigen-binding fragment comprises a sequence set forth in SEQ ID NO: 45, a sequence having at least 90% identity to the sequence set forth in SEQ ID NO: 45, or an amino acid sequence having one or more conservative amino acid substitutions as compared to the sequence set forth in SEQ ID NO: 45.

5. The antibody or the antigen-binding fragment according to claim 4, wherein the heavy chain variable region of the antibody or the antigen-binding fragment comprises a sequence set forth in any one of SEQ ID NOs: 46-49 or 107, a sequence having at least 80% identity to the sequence set forth in any one of SEQ ID NOs: 46-49 or 107, or an amino acid sequence having one or more conservative amino acid substitutions as compared to the sequence set forth in any one of SEQ ID NOs: 46-49 or 107; and/or
a light chain variable region of the antibody or the antigen-binding fragment comprises a sequence set forth in SEQ ID NO: 50, a sequence having at least 80% identity to the sequence set forth in SEQ ID NO: 50, or an amino acid sequence having one or more conservative amino acid substitutions as compared to the sequence set forth in SEQ ID NO: 50.

6. The antibody or the antigen-binding fragment according to claim 5, wherein the antibody or the antigen-binding fragment comprises the heavy chain variable region having an amino acid sequence set forth in any one of SEQ ID NOs: 46-49 or 107 and/or the light chain variable region having an amino acid sequence set forth in SEQ ID NO 50.

7. The antibody or the antigen-binding fragment according to any one of claims 1-6, wherein a heavy chain constant region of the antibody or the antigen-binding fragment comprises a sequence having an amino acid sequence set forth in SEQ ID NO: 51, a sequence having at least 80% identity to the sequence set forth in SEQ ID NO: 51, or an amino acid sequence having one or more conservative amino acid substitutions as compared to the sequence set forth in SEQ ID NO: 51; and/or
a light chain constant region of the antibody or the antigen-binding fragment comprises a sequence having an amino acid sequence set forth in SEQ ID NO: 52, a sequence having at least 80% identity to the sequence set forth in SEQ ID NO: 52, or an amino acid sequence having one or more conservative amino acid substitutions as compared to the sequence set forth in SEQ ID NO: 52.

8. The antibody or the antigen-binding fragment according to any one of claims 1-6, wherein a heavy chain of the antibody comprises a sequence having an amino acid sequence set forth in any one of SEQ ID NOs: 53-56 and 108, a sequence having at least 80% identity to the sequence set forth in any one of SEQ ID NOs: 53-56 and 108, or an amino acid sequence having one or more conservative amino acid substitutions as compared to the sequence set forth in any one of SEQ ID NOs: 53-56 and 108; and/or
a light chain of the antibody comprises a sequence having an amino acid sequence set forth in SEQ ID NO: 57, a sequence having at least 80% identity to the sequence set forth in SEQ ID NO: 57, or an amino acid sequence having one or more conservative amino acid substitutions as compared to the sequence set forth in SEQ ID NO: 57.

9. A nucleic acid molecule encoding the antibody or the antigen-binding fragment according to any one of claims 1-8.

10. A pharmaceutical composition, comprising the antibody or the antigen-binding fragment according to any one of claims 1-8 and a pharmaceutically acceptable carrier.

11. A fusion protein, comprising an antibody or an antigen-binding fragment and a 6-HB interference polypeptide for interfering with fusion of a viral coat with a cell membrane, wherein the antibody or the antigen-binding fragment specifically binds to a spike protein of SARS-CoV-2 or SARS-CoV, and the antibody or the antigen-binding fragment at least comprises one or more of a VH CDR1 set forth in SEQ ID NO: 1, a VH CDR2 set forth in SEQ ID NO: 2, a VH CDR3 set forth in any one of SEQ ID NOs: 3-38 or 105, a VL CDR1 set forth in SEQ ID NO: 39, a VL CDR2 set forth in SEQ ID NO: 40, and a VL CDR3 set forth in SEQ ID NO: 41;
a C-terminus or an N-terminus of the antibody or the antigen-binding fragment is linked to the 6-HB interference polypeptides via a linker, and the linker is a polypeptide comprising glycine and serine.

12. The fusion protein according to claim 11, wherein the fusion protein comprises the following characteristics:
the antibody or the antigen-binding fragment at least comprises the VH CDR1 set forth in SEQ ID NO: 1, the VH CDR2 set forth in SEQ ID NO: 2, the VH CDR3 set forth in any one of SEQ ID NOs: 3-38 or 105, the VL CDR1 set forth in SEQ ID NO: 39, the VL CDR2 set forth in SEQ ID NO: 40, and the VL CDR3 set forth in SEQ ID NO: 41; and/or
the C-terminus of the antibody or the antigen-binding fragment is linked to the 6-HB interference polypeptides via a linker, and the C-terminus of the antibody or the antigen-binding fragment is a C-terminus of the heavy chain or a C-terminus of the light chain of the antibody or the antigen-binding fragment; and/or
a sequence of the linker is (GGGGS)ₙ, and the n is 1, 2, 3, 4 or 5; and/or
the 6-HB interference polypeptide comprises a sequence set forth in SEQ ID NO: 58, a sequence having at least 90% identity to the sequence set forth in SEQ ID NO: 58, or an amino acid sequence having one or more conservative amino acid substitutions as compared to the sequence set forth in SEQ ID NO: 58.

13. The fusion protein according to claim 12, wherein the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 4, the VL CDR1 set forth in SEQ ID NO: 39, the VL CDR2 set forth in SEQ ID NO: 40, and the VL CDR3 set forth in SEQ ID NO: 41; or
the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 10, the VL CDR1 set forth in SEQ ID NO: 39, the VL CDR2 set forth in SEQ ID NO: 40, and the VL CDR3 set forth in SEQ ID NO: 41; or
the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 12, the VL CDR1 set forth in SEQ ID NO: 39, the VL CDR2 set forth in SEQ ID NO: 40, and the VL CDR3 set forth in SEQ ID NO: 41; or
the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 14, the VL CDR1 set forth in SEQ ID NO: 39, the VL CDR2 set forth in SEQ ID NO: 40, and the VL CDR3 set forth in SEQ ID NO: 41; or
the antibody or the antigen-binding fragment at least comprises the VH CDR1 set forth in SEQ ID NO: 1, the VH CDR2 set forth in SEQ ID NO: 2, the VH CDR3 set forth in SEQ ID NO: 105, the VL CDR1 set forth in SEQ ID NO: 39, the VL CDR2 set forth in SEQ ID NO: 40, and the VL CDR3 set forth in SEQ ID NO: 41.

14. The fusion protein according to any one of claims 11-13, wherein framework regions of a heavy chain variable region of the antibody or the antigen-binding fragment comprise a heavy chain FR1, a heavy chain FR2, a heavy chain FR3 and a heavy chain FR4, and the heavy chain FR1 of the antibody or the antigen-binding fragment comprises a sequence set forth in SEQ ID NO: 42, a sequence having at least 90% identity to the sequence set forth in SEQ ID NO: 42, or an amino acid sequence having one or more conservative amino acid substitutions as compared to the sequence set forth in SEQ ID NO: 42; and/or
the heavy chain FR2 of the antibody or the antigen-binding fragment comprises a sequence set forth in SEQ ID NO: 43, a sequence having at least 90% identity to the sequence set forth in SEQ ID NO: 43, or an amino acid sequence having one or more conservative amino acid substitutions as compared to the sequence set forth in SEQ ID NO: 43; and/or
the heavy chain FR3 of the antibody or the antigen-binding fragment comprises a sequence set forth in SEQ ID NO: 44, a sequence having at least 90% identity to the sequence set forth in SEQ ID NO: 44, or an amino acid sequence having one or more conservative amino acid substitutions as compared to the sequence set forth in SEQ ID NO: 44; and/or
the heavy chain FR4 of the antibody or the antigen-binding fragment comprises a sequence set forth in SEQ ID NO: 45, a sequence having at least 90% identity to the sequence set forth in SEQ ID NO: 45, or an amino acid sequence having one or more conservative amino acid substitutions as compared to the sequence set forth in SEQ ID NO: 45.

15. The fusion protein according to claim 14, wherein the heavy chain variable region of the antibody or the antigen-binding fragment comprises a sequence set forth in any one of SEQ ID NOs: 46-49 or 107, a sequence having at least 80% identity to the sequence set forth in any one of SEQ ID NOs: 46-49 or 107, or an amino acid sequence having one or more conservative amino acid substitutions as compared to the sequence set forth in any one of SEQ ID NOs: 46-49 or 107; and/or
a light chain variable region of the antibody or the antigen-binding fragment comprises a sequence set forth in SEQ ID NO: 50, a sequence having at least 80% identity to the sequence set forth in SEQ ID NO: 50, or an amino acid sequence having one or more conservative amino acid substitutions as compared to the sequence set forth in SEQ ID NO: 50.

16. The fusion protein according to claim 15, wherein the antibody or the antigen-binding fragment comprises the heavy chain variable region having an amino acid sequence set forth in any one of SEQ ID NOs: 46-49 or 107 and/or the light chain variable region having an amino acid sequence set forth in SEQ ID NO 50.

17. The fusion protein according to any one of claims 11-16, wherein a heavy chain constant region of the antibody or the antigen-binding fragment comprises a sequence having an amino acid sequence set forth in SEQ ID NO: 51 or a sequence of amino acids at positions 1-328 of SEQ ID NO: 51, or a sequence having at least 80% identity to the sequence set forth in SEQ ID NO: 51 or the sequence of amino acids at positions 1-328 of SEQ ID NO: 51, or an amino acid sequence having one or more conservative amino acid substitutions as compared to the sequence set forth in SEQ ID NO: 51 or the sequence of amino acids at positions 1-328 of SEQ ID NO: 51; and/or
a light chain constant region of the antibody or the antigen-binding fragment comprises a sequence having an amino acid sequence set forth in SEQ ID NO: 52, a sequence having at least 80% identity to the sequence set forth in SEQ ID NO: 52, or an amino acid sequence having one or more conservative amino acid substitutions as compared to the sequence set forth in SEQ ID NO: 52.

18. The fusion protein according to any one of claims 11-16, wherein the fusion protein comprises the following characteristics:
a heavy chain of the antibody comprises a sequence having an amino acid sequence set forth in any one of SEQ ID NOs: 53-56 and 108 or a sequence of amino acids at positions 1-450 of any one of SEQ ID NOs: 53-56 and 108, or a sequence having at least 80% identity to the sequence set forth in any one of SEQ ID NOs: 53-56 and 108 or the sequence of amino acids at positions 1-450 of any one of SEQ ID NOs: 53-56 and 108, or an amino acid sequence having one or more conservative amino acid substitutions as compared to the sequence set forth in any one of SEQ ID NOs: 53-56 and 108 or the sequence of amino acids at positions 1-450 of any one of SEQ ID NOs: 53-56 and 108; and/or
a light chain of the antibody comprises a sequence having an amino acid sequence set forth in SEQ ID NO: 57, a sequence having at least 80% identity to the sequence set forth in SEQ ID NO: 57, or an amino acid sequence having one or more conservative amino acid substitutions as compared to the sequence set forth in SEQ ID NO: 57; and/or
a C-terminus of the heavy chain or a C-terminus of the light chain of the antibody is covalently linked, via a linker set forth in SEQ ID NO: 59, to the 6-HB interference polypeptide, wherein the 6-HB interference polypeptide comprises a sequence set forth in SEQ ID NO: 58, a sequence having at least 90% identity to the sequence set forth in SEQ ID NO: 58, or an amino acid sequence having one or more conservative amino acid substitutions as compared to the sequence set forth in SEQ ID NO: 58.

19. The fusion protein according to claim 18, wherein the fusion protein comprises the following characteristics:
the heavy chain of the antibody comprises a sequence having an amino acid sequence of amino acids at positions 1-450 of SEQ ID NO: 108, and the light chain of the antibody comprises a sequence having an amino acid sequence set forth in SEQ ID NO: 57;
the C-terminus of the heavy chain of the antibody is covalently linked, via the linker set forth in SEQ ID NO 59, to the 6-HB interference polypeptide, wherein the 6-HB interference polypeptide comprises the sequence set forth in SEQ ID NO 58.

20. A nucleic acid molecule encoding the fusion protein according to any one of claims 11-19.

21. A pharmaceutical composition, comprising the fusion protein according to any one of claims 11-19 and a pharmaceutically acceptable carrier.
